(19)

**Europäisches Patentamt**

**European Patent Office**

**Office européen des brevets**

(11) **EP 0 444 115 B2**

(12) **NEW EUROPEAN PATENT SPECIFICATION**

(45) Date of publication and mention
of the opposition decision:
**13.12.2000 Bulletin 2000/50**

(45) Mention of the grant of the patent:
**28.08.1996 Bulletin 1996/35**

(21) Application number: **89913278.1**

(22) Date of filing: **15.11.1989**

(51) Int. Cl.[7]: **C12Q 1/68**

(86) International application number:
**PCT/US89/05253**

(87) International publication number:
**WO 90/05789 (31.05.1990 Gazette 1990/12)**

(54) **IN SITU SUPPRESSION HYBRIDIZATION AND USES THEREFOR**

IN-SITU-UNTERDRÜCKUNGS-HYBRIDISIERUNG UND VERWENDUNGEN

HYBRIDATION PAR SUPPRESSION IN SITU ET UTILISATIONS

(84) Designated Contracting States:
**AT BE CH DE FR GB IT LI LU NL SE**

(30) Priority: **15.11.1988 US 271609**

(43) Date of publication of application:
**04.09.1991 Bulletin 1991/36**

(73) Proprietor: **YALE UNIVERSITY**
**New Haven, CT 06510 (US)**

(72) Inventors:
• **WARD, David, C.**
**Guilford, CT 06437 (US)**
• **LICHTER, Peter**
**D-6900 Heidelberg (DE)**
• **CREMER, Thomas**
**D-6900 Heidelberg (DE)**
• **MANUELIDIS, Laura**
**New Haven, CT 06511 (US)**

(74) Representative:
**Holdcroft, James Gerald, Dr. et al**
**Graham Watt & Co.,**
**Riverhead**
**Sevenoaks, Kent TN13 2BN (GB)**

(56) References cited:
**GB-A- 2 215 724**

• **Cancer Research, Vol. 48, 1988 Peter Devilee et al: "Detection of Chromosome Aneuploidy in Interphase Nuclei from Human Primary Breast Tumors Using Chromosome-specific Repetitive DNA Probes ", page 5825 - 5830**
• **American journal of human genetics., Vol. 43, No. 3, 1988 D. Pinkel et al: "Detection of structural chromosome abberations in metaphase spreads and interphase nuclei by in situ hybridization high complexity probes which stain entire human chromosomes ", Abstract no. 0471, page A 118**
• **Experimental Cell Research, Vol. 176, 1988 T. Cremer et al: "Rapid Interphase and Metaphase Assessment of Specific Chromosomal Changes in Neuroectodermal Tumor Cells by in Situ Hybridization with Chemically Modified DNA Probes ", page 199 - 202**
• **Human Genetics, Vol. 74, 1986 T. Cremer et al: "Detection of chromosome aberrations in the human interphase nucleus by visualization of specific target DNAs with radioactive and non-radioactive in situ hybridization techniques: diagnosis of trisomy 18 with probe L1.84 ", page 346 - 352**
• **Molec Cell Biol 3 (1983) 2076**
• **Am J Hum Genet 43 (1988) Suppl, Abs No.(0471) 11.5**
• **Research grant application from Lawrence Livermore National Laboratory to NIH; cover letter dated 060386**
• **Europ J Cell Biol 44 (1987) Suppl 20, Abs No 97**
• **Proc Natl Acad Sci USA 83 (1986) 2934**
• **"In situ hybridization to cellular RNAs" in Genetic Engineering, Principles and methods 7 (1985) 43 (Plenum Press, NY)**

- **Molecular Cloning, Sambrook et al, 2nd Edition (1989) p9.49**
- **Hum Genet 77 (1987) 366**
- **Hum Genet 67 (1984) 317**
- **Hum Genet 72 (1986) 86**
- **Proc Natl Acad Sci USA (9186) 4408**
- **Workshop Schedule for XIVth International Congress of Genetics**
- **Exp Cell Res 153 (1984) 61**
- **Cell 52 (1988) 51**

## Description

### Background

Chromosome banding techniques have facilitated the identification of specific human chromosomes and presently provide the major basis upon which chromosomal aberrations are diagnosed. The interpretation of chromosome banding patterns requires skilled personnel and is often technically difficult, especially with respect to detecting minor structural changes and when analyzing complex karyotypes, such as those of highly aneuploid tumor cells. An additional complexity is that readable metaphase chromosome spreads are sometimes very difficult or impossible to prepare from certain cell types or tissues. Alternative methods for identifying chromosomal aberrations would be valuable because they could augment current methods of cytogenic analysis, particularly if such alternative methods were applicable to both mitotic and interphase cell populations.

Over the past few years, a considerable body of evidence has been obtained which indicates that the DNA of individual chromosomes occupy focal territories, or spatially cohesive domains, within mammalian interphase nuclei. Cremer, T. et al., Hum. Genet., 60:66-56 (1982); Hens, L. et al., Exp. Cell Res., 149:257-269 (1983); Schardin, M. et al., Hum. Genet., 71:281-287 (1985); Manuelidis, L., Hum Genet., 71:288-293 (1985); and Pinkel, D. et al., Proc. Natl. Acad. Sci. USA, 83:2934-2938 (1986). These observations suggest that chromosome-specific probe sets could be used to detect numerical or structural aberrations of chromosomal domains in non-mitotic cells, an approach termed "interphase cytogenics". Cremer, T. et al., Hum. Genet., 74:346-352 (1986). Indeed, recent in situ hybridization studies have demonstrated the prenatal diagnosis of trisomy-18 with interphase cells and the detection of numerical chromosomal abnormalities in tumor cells lines using chromosome-specific repetitive DNAs as probes. Cremer, T. et al., Hum. Genet., 74:346-352 (1986) and Cremer, T. et al., Exp. Cell Res., 176:119-220 (1988). All chromosome-specific repetitive DNAs reported to date are localized to discrete subregions of each chromosome and, thus, such DNA probes are unsuitable for analyses of many types of chomosomal aberrations (e.g., translocations and deletions). If it were possible to detect uniquely the spectrum of sequences comprising a specific chromosome, analysis of aberrations of chromosomal domains in non-mitotic cells would be possible. Furthermore, such a general labeling technique would make it possible to address fundamental questions concerning the spatial organization of chromosomal DNA within interphase nuclei.

### Disclosure of the Invention

The subject invention relates to a method of detecting, identifying and/or quantitating selected individual chromsomes in mammalian mitotic or interphase cells, by means of chromosomal in situ suppression (CISS) hybridization and its use in analyzing cells for the occurrence of chromosomes, chromosome fragments, or chromosome aberrations, such as those associated with a condition or disease. In the method of the present invention, chromosome-specific probes (DNA or RNA) are combined with a sample to be analyzed, in such a manner that an individual chromosome(s) of interest is labeled and the complex spectrum of sequences which comprise the chromosome can be detected. The probes used in the present method are of high genetic complexity and can be appropriately-selected cloned DNA or RNA fragments, used individually or in pools, or chromosome library DNA.

The method of the present invention, referred to as CISS hybridization, is particularly useful because it can be used to specifically stain individual mammalian chromosomes at any point in the cell cycle. It can be used to assess chromosomal content, particularly chromosome aberrations (e.g., deletions, rearrangements, change in chromosome number) which, until the present invention, it has been time-consuming and/or difficult, if not impossible, to detect. The method is useful in providing a rapid and highly specific assessment of individual mammalian chromosomes in any context (e.g., diagnosis and/or monitoring of a genetic condition or a disease state) in which such an assessment is desired.

### Brief Description of the Drawings

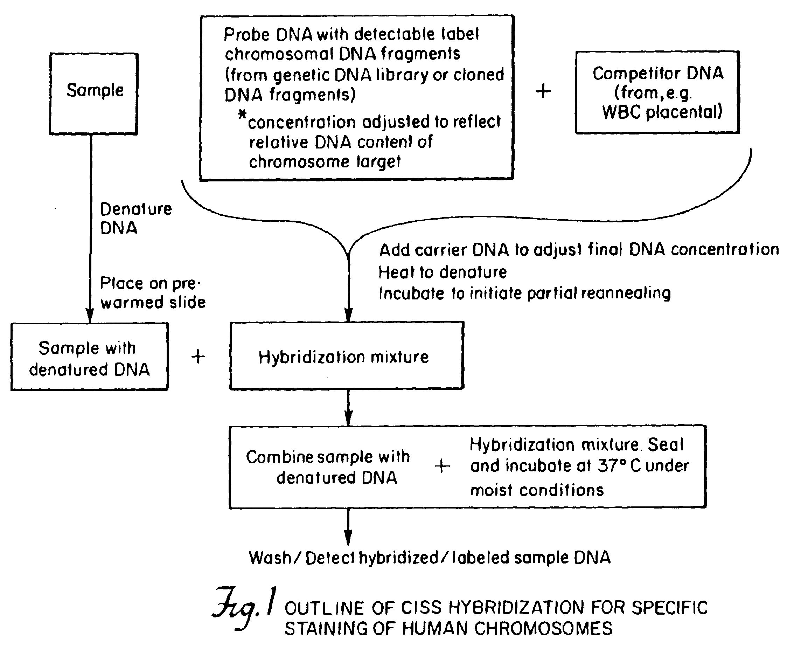

Figure 1 presents an outline of the CISS hybridization method of the present invention.

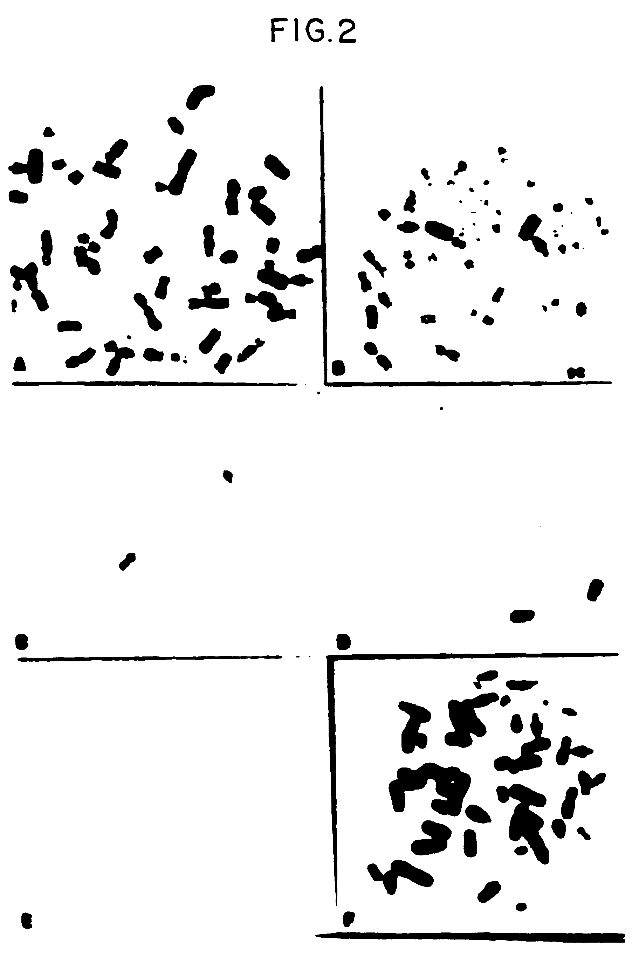

Figure 2 shows suppression from cross-reacting sequences within a chromosome 7-derived DNA library by different concentrations of human competitor DNA. Biotin-labeled chromosome 7 DNA inserts (20 µg/ml) were prehybridized for 20 minutes with human genomic DNA prior to hybridization with metaphase chromosome spreads and detection with FITC-labeled avidin. Human DNA concentrations were: A, 0 µg/ml; B, 50 µg/ml; C, 100 µg/ml; D, 200 µg/ml; E, 1000 µg/ml; F, same metaphase spread as in E post-stained with DAPI. Genomic salmon DNA was added to each sample to adjust the final DNA concentration to 1.0 mg/ml (see the text for details). The arrows mark the target chromosome 7 and the arrowheads mark additional strong signals on non-7 chromosomes. All negatives printed were exposed and developed under identical photographic conditions.

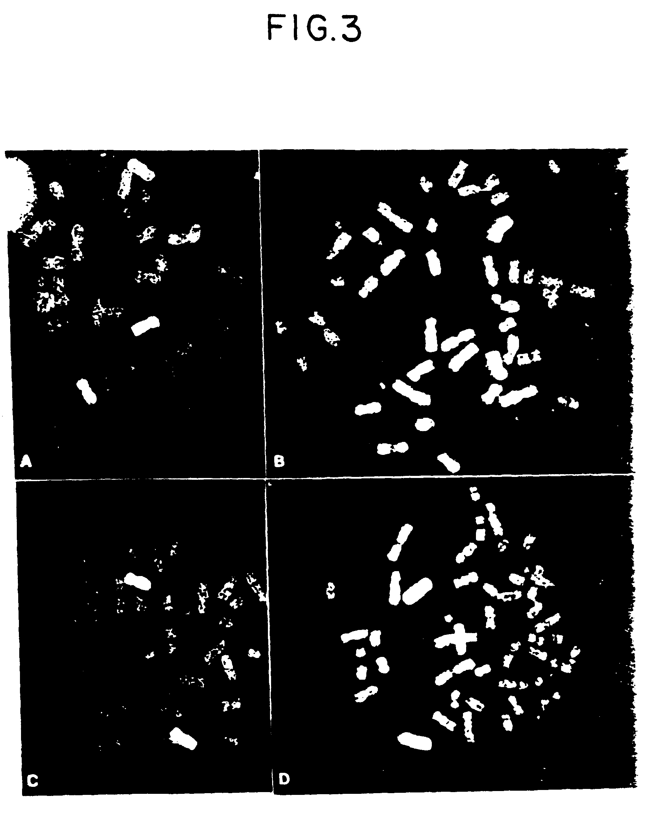

Figure 3 shows the effect of pre-annealing time on the specificity and strength of the hybridization signal. Biotin-labeled chromosome 7 DNA inserts (20 μg/ml) were preannealed with 200 μg/ml human competitor DNA for the following times, prior to hybridization to metaphase A, 0 minutes; B, 2 minutes; C, 5 minutes; D, 20 minutes.

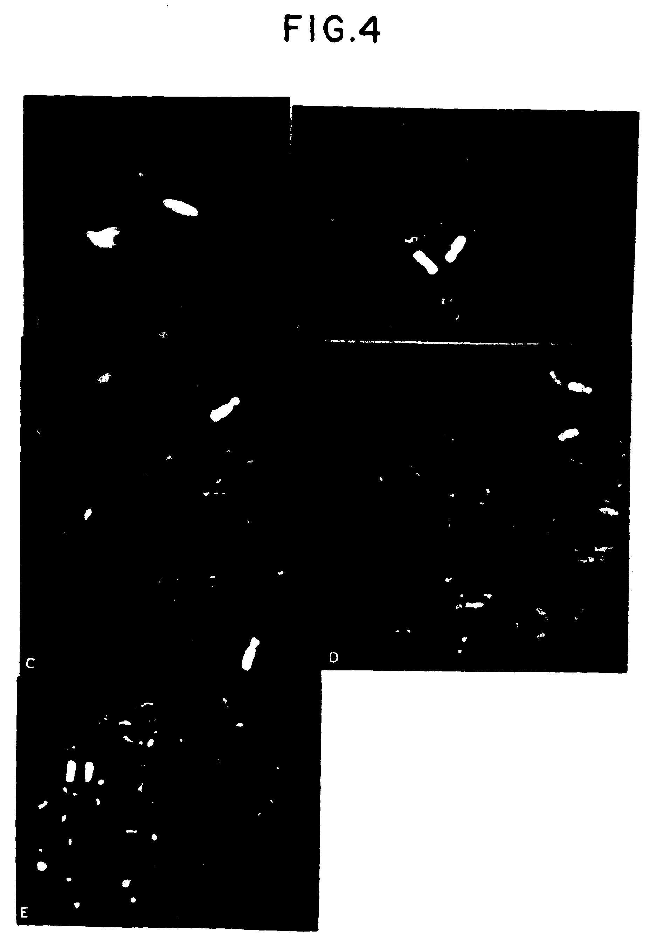

Figure 4 shows decoration of A chromosome 1, B chromosome 7, C chromosome 4, D chromosome 18, E chromosome 13 and F chromosome 20 in normal human lymphocytes. Only the chromosome 13 insert DNA pool shows significant cross-hybridization to other chromosomes after the prehybridization suppression step. The detection of chromosome 20 (F) was done with the entire chromosome library (including λ phage arms) and detected with avidin-alkaline phosphatase using nitro blue tetrazolium/5-bromo-4-chloro-3-indolyl phosphate (NBT-BCIP) as the enzyme substrate mixture. The signal of chromosome 1 (A) was amplified by the sandwich technique of Pinkel et al. (1986).

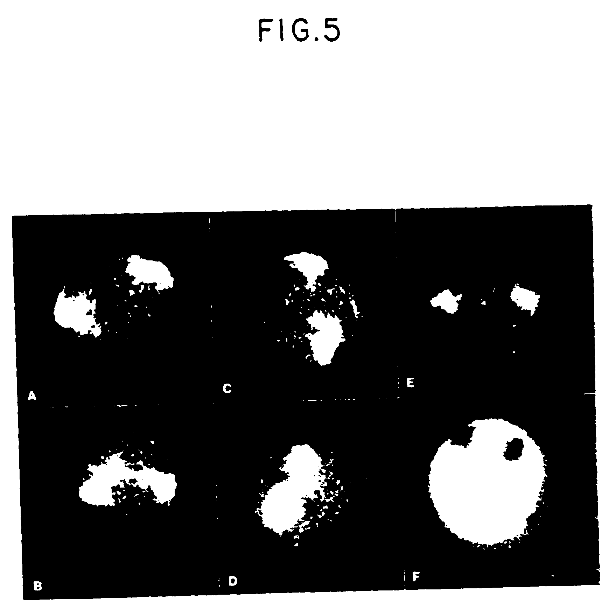

Figure 5 shows chromosome domains in human lymphocyte nuclei delineated by preannealed chromosome library DNA inserts. Hybridization to acetic acid-methanol fixed nuclei was detected by fluorescein isothiocyanate (FITC)-conjugated avidin (A-E) or alkaline phosphatase-conjugated avidin (F). Domains are shown for chromosome 1 (A,B), chromosome 7 (C,D) and chromosome 18 (E,F). A predominant staining of the centromere region is seen within the chromosome 7 domains, reflecting preferential hybridization of the chromosome 7-specific alphoid DNA repeat; a similar signal distribution on metaphase chromosomes was also observed in this particulate experiment.

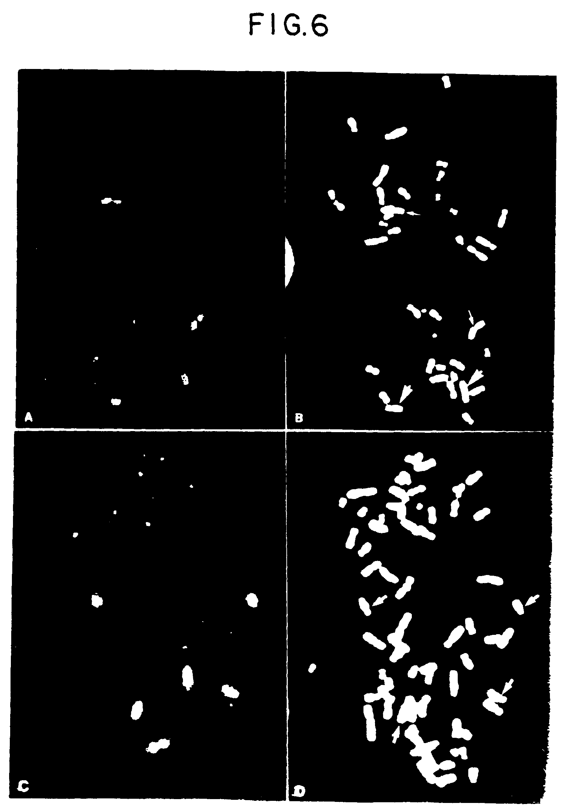

Figure 6 shows chromosomal in situ suppression (CISS) hybridization of chromosome 1 inserts to metaphase spreads of glioma cell lines detected with avidin-FITC (A,C) and poststained with 4,6-diamidino-2-phenylindole dihydrochloride (DAPI) (B,D). A,B TC 620 show two apparently complete 1 chromosomes (small arrows in B) and two marker translocation chromosomes (arrowheads) specifically decorated by these inserts (A). One of the two marker chromosomes contains a 1p (lower left), the other a 1q arm (lower right); the 1p terminal (relatively GC rich region) in the two normal chromosomes and submetacentric marker is less completely delineated. Also, the 1q12 regions here show little decoration in contrast to most experiments. X 950. C,D Typical TC 593 metaphase spreads show six specifically decorated chromosomes. Three acrocentric marker chromosomes all with truncation of 1p show particularly intense fluorescence of repeats that localize to 1q12 (arrows in C). In two of these, 1q arms appear to be complete, while a major deletion is obvious in the third (the arrow in D). A fourth decorated chromosome (small arrowhead in C,D) again shows a major deletion of the distal part of 1q, but has retained an apparently complete 1p arm. A fifth submetacentric chromosome (large arrowhead) contains an apparently complete 1p arm; the DNA of its short arm has not been identified. Note the similarity of this marker to one of the marker chromosomes of TC 620 (1p) described above. The sixth entirely decorated chromosome is an iso (1p) as demonstrated by DAPI-banding (open arrows). X 1200

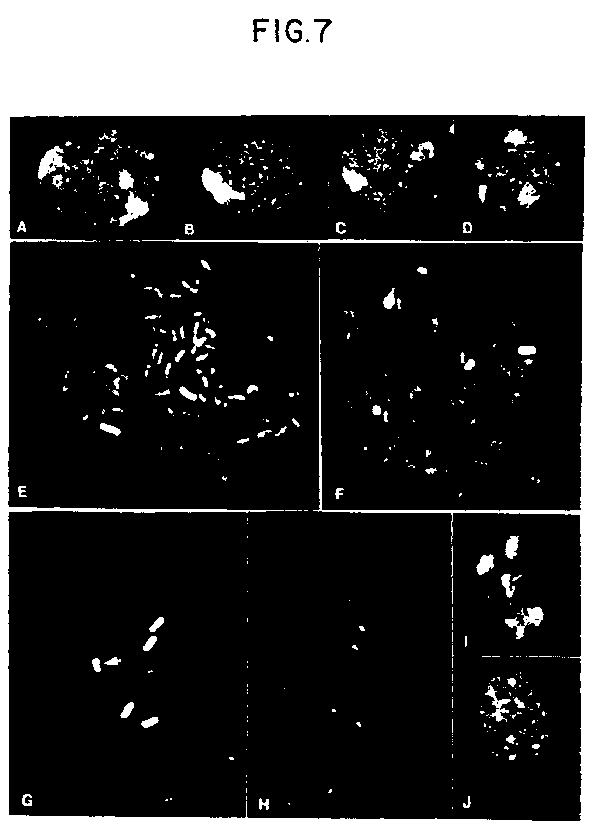

Figure 7 shows CISS hybridization of chromosome 4 library inserts detected with a avidin-FITC; interphase nuclei of TC 593 (A-C) and TC 620 (D). Note that the two apparently complete interphase domains are close to each other in B, but widely separated in A and C. TC 620 interphase nucleus (D) shows four chromosome 4 interphase domains of largely different sizes. Metaphase spread of TC 593 (E) shows two apparently complete 4 chromosomes, and a small decorated region (arrow) in a submetacentric chromosome. This marker with translocated 4 sequences was observed in about 30% of the spreads. TC 620 metaphase spread (F) shows one apparently complete chromosome 4 and three translocation markers (t) containing different amounts of chromosome 4 material. G-J Double hybridization of biotinylated chromosome 7 inserts and an aminoacetylfluorene (AAF)-modified 7-specific alphoid repeat. G Chromosome 7 inserts depict five entirely decorated metaphase chromosomes. Four of them are complete 7 chromosomes, the fifth (arrow) is an iso (7p) (see Fig. 3E). H The same field as G showing AAF-7 alphoid signals on only four decorated chromosomes; no signal is detected on the iso (7p). I An interphase nucleus of TC 593 shows five domains delineated by chromosome 7 inserts; four of these are labeled by 7 alphoid probes (J). The arrow in I represents the iso (7p) marker in interphase.

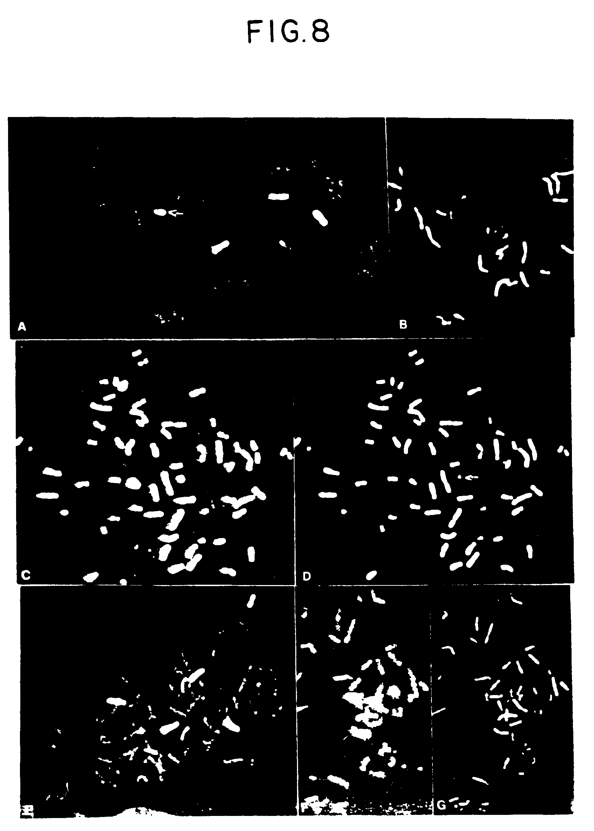

Figure 8 shows CISS hybridization of library inserts of chromosomes 7 and 18 to metaphase spreads of glioma cell lines detected with avidin-FITC. X 875. A,B TC 620 hybridized to chromosome 7 inserts. Three apparently normal 7 chromosomes and an additional translocation chromosome containing 7 sequences are indicated by large arrowhead in A; DAPI-stained complete chromosomes are indicated by the small arrows in B. Other studies (see the text) indicated a translocation of 7pter-q11 in the marker chromosome (large arrowhead). D, The corresponding frame counterstained with DAPI. E, Metaphase spread from pseudotetraploid TC 593 shows five chromosomes highlighted by 7 library inserts. The metacentric chromosome (m) represents the iso (7p) marker typical for this line (see also Fig. 2G). Insert chromosomes (small arrows) show DAPI-stained normal and metacentric 7 chromosomes. The landmark band 7q21 and a block of constitutive heterochromatin at 7q11 are both prominent on the normal chromosome 7 insert, (arrows) but not present on the marker chromosome. Instead both arms of the latter show a mirror-like staining pattern with a faint distal band at 7p21. F,G TC 593 hybridized to chromosome 18 inserts. Four decorated 18 chromosomes are shown in F and three of them in G by DAPI staining are clearly translocated (t).

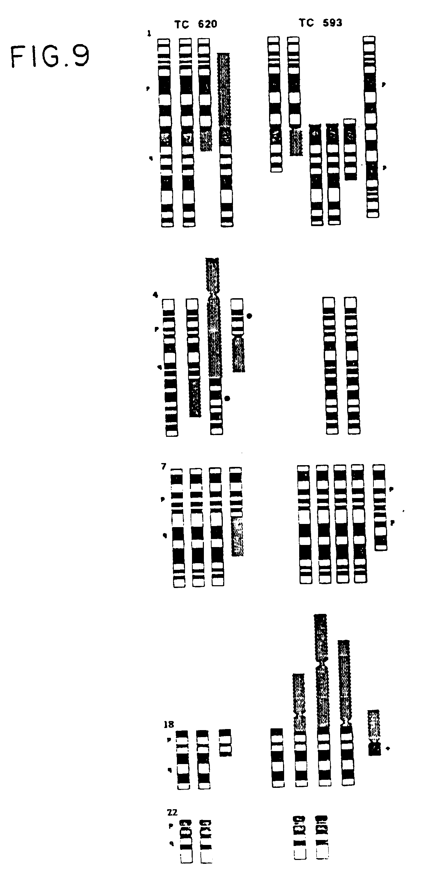

Figure 9 is a summary chromosome idiogram of complete and aberrant chromosomes detected by CISS hybridization of library inserts of chromosomes 1, 4, 7, 18 and 20 in glioma cell lines TC 620 (left) and TC 593 (right). G-bands (black) are shown with approximate breakpoints suggested by our data; the shaded areas with a wavy pattern are from other chromosomes that constitute part of the marker translocation chromosomes. The black dot beside two of the TC 620 translocated 4 segments indicates that the assignment of the chromosome 4 material is based on circumstantial evidence (e.g., size measurements). A small translocation of chromosome 18 material in ca. 20% of TC 593 metaphase spreads (+) also could not be further identified. Note the over-representation of 7p in both cell lines.

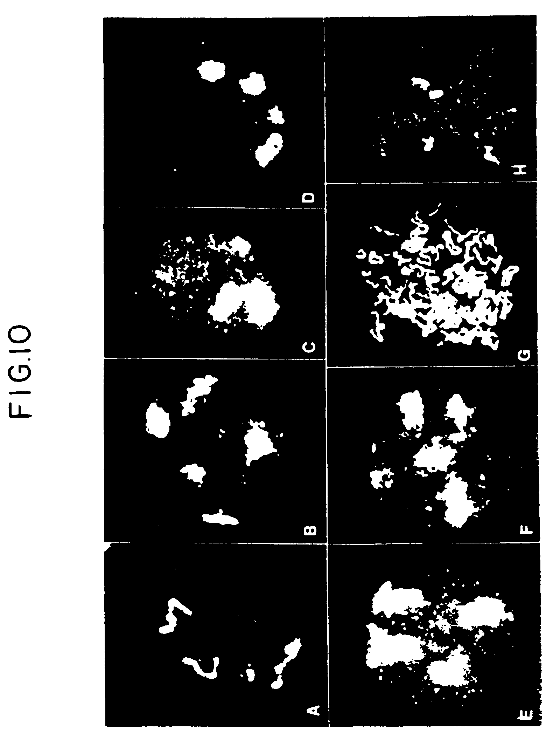

Figure 10 shows representative prophase (A) and interphase nuclei (B-F) reflect metaphase abnormalities in glioma lines (cf. Figs. 1-3). A, Detection of the 7p translocation (t) in a prophase TC 620 nucleus. X 1,000. B, Five well-separate chromosome 7 domains detected in TC 593. X 1,240. C, Two large and one very small 18 domains (indicated by arrow head) in TC 620. X 1,450. D, Four chromosome 18 domains detected in TC 593; one of these signals (arrow) appears smaller. X 1,450. E, Four chromosomal 1 domains in TC 620 (cf. Fig. 1A, B). X 1,200. F, At least five chromosome 1 domains in TC 593; one (arrow head) is appreciably smaller than the others. X 1,250. G,H, CISS hybridization of a technically poor metaphase spread of TC 593 poststained with DAPI (G) still highlights four distinct chromosomes bearing 18 sequences (H). X 1,000.

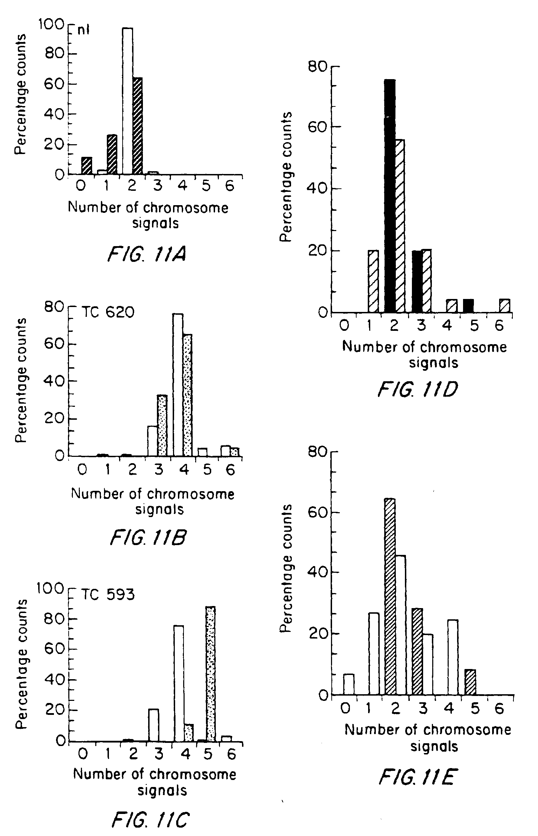

Figure 11 is a graphic representation of the interphase and/or metaphase counts of chromosomes 7 (A-C), 22 (D) and 4 (E) by CISS hybridization. Interphase counts were performed on 150 nuclei of well-hybridized preparations. For metaphase counts > 25 complete DAPI-stained spreads were evaluated. A-C Counts of 7 specific alphoid repeats (white columns) compared to 7 library inserts (shaded columns): A, interphase nuclei of phytohemagglutinin-stimulated human lymphocytes (46,XY); B, TC 620 interphase nuclei (7-specific alphoid repeat) and metaphase spreads (7 library inserts); C, TC 593 interphase nuclei (7-specific alphoid repeat) and metaphase spreads (7 library inserts). High stringency hybridization (see Materials and methods) of 7 alphoid repeat was used to avoid cross-hybridization to other chromosomes. In cases of double hybridization with both 7 library inserts and alphoid repeat (shown in Fig. 2 G-I) standard conditions with 50% formamide were sufficient to avoid cross-hybridization, possibly due to the presence of human competitor DNA. D, Counts of chromosome 22 (library inserts) in metaphase spreads of TC 620 (black columns) and TC 593 (shaded columns). For comparison, CISS hybridization was simultaneously performed with 7 library inserts in these experiments as an internal control (see C,D and Fig. 7). E, Interphase counts (white columns) and metaphase counts (shaded columns) compared in TC 593 hybridized with chromosome 4 inserts. Note the ratio of signal preparations 2:3 are the same in metaphase and interphase.

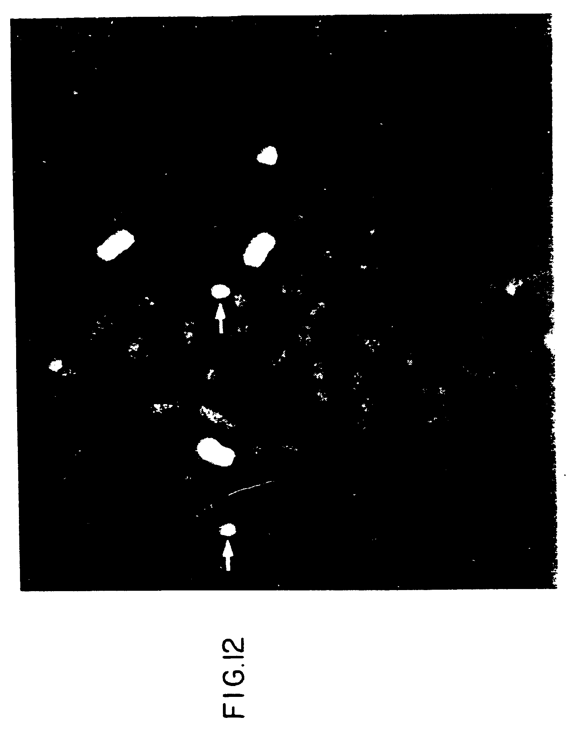

Figure 12 shows a TC 620 metaphase spread after double hybridization with inserts from chromosomes 7 and 22 (both labeled with biotin and detected with avidin-FITC). Two strongly decorated 22 chromosomes (arrows), three complete 7 chromosomes, and the metacentric marker chromosome containing 7pter-q11 are also seen.

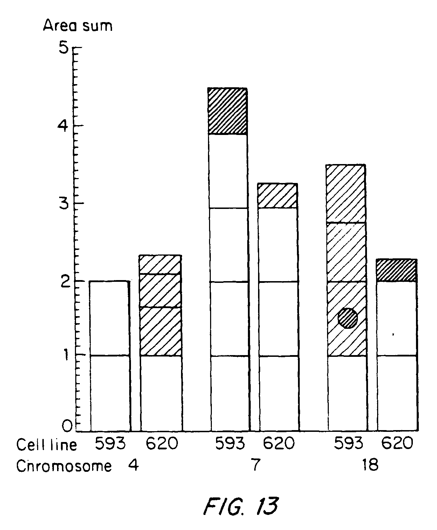

Figure 13 is a graphic representation of the relative size of decorated normal and aberrant chromosomes 4, 7 and 18 in typical metaphase spreads (n-24) from glioma cell lines TC 593 and TC 620. Individual areas were normalized so that a complete chromosome is represented by an area of 1 (see legend to Table 1). The total added signals reflect the number of specific chromosome equivalents present. The white regions correspond to apparently normal chromosomes, the black regions indicate small free chromosome segments entirely decorated by specific library inserts, and translocated segments are shaded. One of the three translocated 18 chromosomes in TC 593 represents a complete chromosome by this measurement (indicated by the black dot), while the two other translocations are slightly smaller, possibly due to the small sample size.

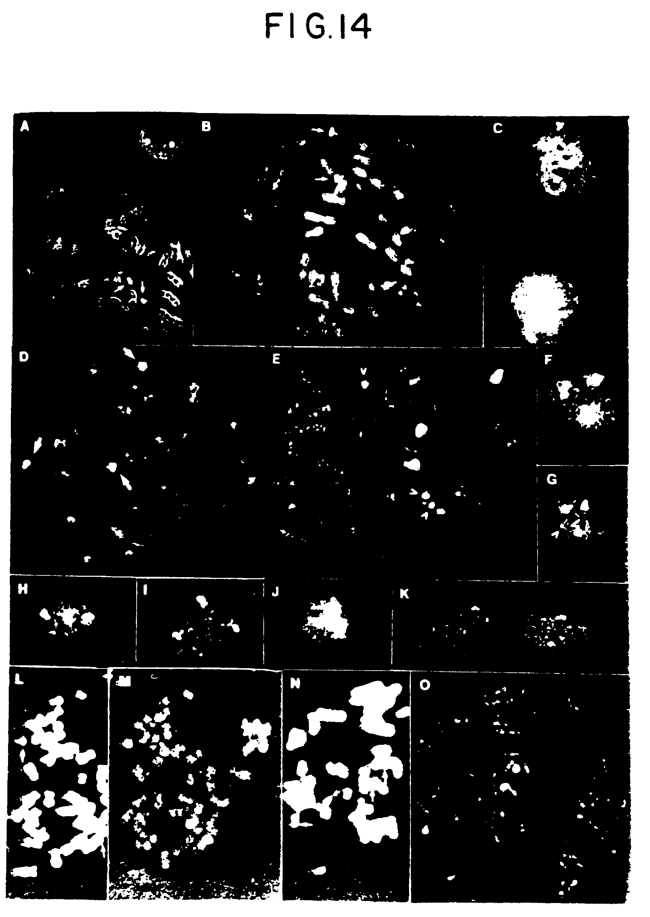

Figure 14 shows specific labeling of human chromosome 21 by CISS hybridization with biotinylated DNA probe sets. A, plasmid pPW519-1R (6-Kb insert) hybridized to a normal lymphocyte metaphase spread. Signals are located on the termini of 21q (see 4',6-diamidino-2-phenylindole (DAPI)-stained chromosomes in Inset) as verified by DAPI banding (not shown). B and C, Normal human lymphocyte metaphase (B) and nuclei (C) after hybridization with the 94 Kb plasmid pool probe set. The terminal band 21q22.3 is specifically labeled. D and E, Signals on trisomy 21 (47, + 21) lymphocyte metaphase spreads after hybridization with the 94 Kb probe set (D) or chromosome 21 library DNA inserts with the CISS hybridization (14) protocol. (E) Three chromosomes 21 are entirely delineated by the library inserts; additional minor signals (see text) are indicated by arrowheads (also in G). (F-J) Labeling of trisomy 21 lymphocyte nuclei by the library inserts (F and G; compare with E).

## Detailed Description of the Invention

The present invention is based on a hybridization strategy in which suppression of hybridization signals from ubiquitous repeated DNA sequences is achieved by using total DNA in a reannealing procedure which is based on rapid reassociation kinetics. The hybridization method of the present invention referred to as chromosomal in situ suppression (CISS) hybridization because of the selective suppression of such signals, has been shown to result in specific cyto-staining of one or more selected individual chromosomes, particularly human chromosomes, at any point in the cell cycle and has been used to detect, identify and quantitate chromosomal aberrations in both mitotic cells and interphase cells (i.e., interphase nuclei).

Described below and in greater detail in the Examples, are the following:

1. specific staining in mitotic and interphase cells of individual human chromosomes, by the method of the present invention (CISS hybridization), using chromosome-specific probe sets which are of high genetic complexity (i.e., chromosome library DNA, cloned DNA fragments);

2. specific staining of metaphase and interphase tumor cells by CISS, using chromosome-specific library probes; and

3. rapid detection in mitotic and interphase cells from a variety of sources of aberrations in a human chromosome (chromosome 21) which is associated with a genetic condition (Down syndrome), using CISS hybridization.

4. demonstration that a nested set of chromosome specific unique sequence probes has been used to identify chromosome aberrations and to detect genetic disease (e.g., Down Syndrome).

Specific Staining of Individual Human Chromosomes

By use of the CISS hybridization method, individual (such as the X and Y chromosomes or homolog pairs of chromosomes 1-22) human chromosomes have been specifically stained in both mitotic and interphase cells. This has been carried out in both metaphase spreads and interphase nuclei and has been used to stain or label one selected (individual) chromosome and to stain or label multiple selected (individual) chromosomes simultaneously, using, respectively, signal-probe CISS hybridization and multi-probe CISS hybridization in conjunction with an appropriate detection method. The method is represented schematically in Figure 1.

Specific Chromosome Staining Using Genomic DNA Libraries and Cloned DNA

CISS hybridization was carried out as follows, to produce specific staining of individual human chromosomes, using commercially-available genomic DNA libraries that originated from flow-cytometry sorted human chromosomes and cloned DNA fragments. Van Dilla, M.A. et al., Biotechnology, 4:537-552 (1986). Suppression of hybridization signals from ubiquitous repeated sequences, such as the Alu and KpnI elements, was achieved using total human DNA in a reannealing procedure that is based on rapid reassociation kinetics. Similar principles have been used by others to facilitate the selective hybridization of unique sequence subsets from cosmid DNA clones for Southern blotting and in situ hybridization experiments. Sealey, P.G. et al., Nucleic Acids, 13:1905-1922 (1985); and Landegent, J.E. et al., Hum. Genet., 77:366-370 (1987). Specific labeling of individual chromosomes in both metaphase spreads and interphase nuclei, is carried out (and shown to have occurred) in the following manner, which is described in detail in the Examples. The feasibility of using computer-assisted optical sectioning for 3-D reconstruction of chromosomal domains for the analysis of nuclear topography was also demonstrated in conjunction with CISS hybridization.

Initially, genomic DNA from a selected chromosome or selected chromosomes is prepared for use as probe DNA. Genomic DNA is available from several sources. For example, one or more genomic DNA libraries, each containing the chromosome of interest (a chromosome-derived library), is used to produce the necessary DNA probes. Such libraries can be commercially-available genomic DNA libraries that originated from flow-cytometry sorted human chromosomes. These are available from the American Type Culture Collection (Rockville, MD). Such DNA libraries for human chromosomes 1, 4, 7, 8, 9, 12, 13, 14, 16, 17, 18, 20, 21, 22 and chromosome X have been used in the present method, as described in the Examples. Other commercially available genomic DNA libraries or genomic DNA libraries from noncommercial sources can also be used. Alternatively, individual plasmid, phage, yeast artificial chromosomes with non-yeast DNA inserts, and cosmid DNA clones can be used as a source of DNA probes for a selected individual chromosome or multiple selected chromosomes. In the case of DNA from a genomic library, the DNA can be separated as a pool from the vector containing it, prior to labeling with a detectable signal, or can be used without separation from the vector.

Probes are labeled with a detectable signal, which can be a fluorescent reporter, one member of a specific binding pair (e.g., biotin-avidin or ligand-antibody), or an enzyme. DNA removed from the vector is labeled by nick translation (using, for example, Bio-11-dUTP), by random primer extension with (e.g., 3' end tailing), for example, the Amersham multiprime DNA labeling system, substituting dTTP with Bio-11-dUTP, or other appropriate technique. In the case of DNA which has not been separated from the vector, biotin labeling is carried out directly by nick translation, using standard techniques. Brigati et al., Virology, 126:32-50 (1983). Other labels can be added in a similar manner (e.g., 2,4-dinitro phenol, digoxin).

Probe size is carefully selected and controlled in order to facilitate probe penetration and to optimize reannealing hybridization. Labeled DNA fragments smaller than 500 nucleotides are used, and, more generally, the majority of the probes are 150-250 nucleotides in length. Probes of this length are made from longer nucleotide sequences using publicly available restriction enzymes or known techniques for producing and recovering appropriately-sized fragments. It is also possible, if the nucleotide sequence of a selected chromosome is known, to synthesize an oligonucleotide having that sequence, using known techniques. Such oligonucleotides, once labeled, can be used to decorate specific chromosomal regions. For example, oligonucleotide probes which specifically hybridize to telomeric sequences of mammalian chromosomes have been identified. Moyuif et al., Proceedings of the National Academy of Sciences, USA, Sept. 1988.

Competitor DNA, which is DNA which acts to suppress hybridization signals from ubiquitous repeated sequences, will be selected as needed (e.g., based on the mammal whose chromosomes are being analyzed). In the case of analysis of human chromosomes, competitor DNA is total human DNA which acts to suppress hybridization from ubiquitous repeated sequences, such as the Alu and the KpnI elements. It is available from many sources. For example, human genomic DNA from placenta or white blood cells can be prepared using known techniques, such as that described by Davis et al. Davis, L.G. et al., Basic methods in molecular biology, Elsevier, N.Y./ Amsterdam (1986). It is digested, using standard methods (e.g., with DNAse), to produce competitor DNA fragments within the same size distribution as the probe DNA.

DNA from another source, which will compete with only a small portion of the human DNA and which is used, as necessary, to adjust the total (final) DNA concentration of the hybridization mixture will also be included, as needed. This DNA is referred to as carrier DNA. This DNA is produced or treated, using standard methods, so that it is within the same size distribution as the probe DNA.

Initially, probe DNA bearing a detectable label and competitor DNA are combined under conditions appropriate for preannealing to occur. The quantity of probe DNA combined with competitor DNA is adjusted to reflect the relative DNA content of the chromosome target. For example, chromosome 1 contains approximately 5.3 times as much DNA as is present in chromosome 21. Probe concentrations were 30 µg/ml and 5 µg/ml, respectively. When total genomic library DNA is used as the probe mixture (instead of purified DNA inserts), approximately 10 times as much labeled DNA is added to compensate for the vector sequences, which are present in large quantities. Only twice as much labeled library DNA is added in the case of the libraries LA0XNL01 (X chromosome) and LA16NL02 (chromosome 16) because the human DNA inserts constitute almost half of the total library DNA. Carrier DNA, such as trout or salmon testis DNA, is added to bring the total DNA concentration to a predetermined level, if necessary. As described herein, sufficient salmon testis DNA was added to result in a final DNA concentration of 1.0 mg/ml in the hybridization mixture (which includes all three types of DNA: probe DNA, competitor DNA and DNA which does not significantly compete).

The resulting hybridization mixture is treated (e.g., by heating) to denature the DNA present and incubated at approximately 37°C for sufficient time to promote partial reannealing.

The sample containing chromosome DNA to be identified (specifically labeled) is also treated to render DNA present in it available for hybridization with complementary sequences, such as by heating to denature the DNA. The hybridization mixture and the sample are combined, under conditions and for sufficient time conducive to hybridization. After sufficient time, detection of specific labeling of the chromosome target is carried out, using standard techniques. For example, as described in the Examples, a biotinylated probe is detected using fluorescein-labeled avidin or avidin-alkaline phosphatase complexes. For fluorochrome detection, samples are incubated, for example, with fluorescein isothiocyanate (FITC)-conjugated avidin DCS (see Example 1). Amplification of the FITC signal can be effected, if necessary, by incubation with biotin-conjugated goat anti-avidin D antibodies, washing and a second incubation with FITC-conjugated avidin. For detection by enzyme activity, samples are incubated, for example, with streptavidin, washed, incubated with biotin-conjugated alkaline phosphatase, washed again and pre-equilibrated (e.g., in AP-buffer, as described in Example 1). The enzyme reaction is carried out in, for example, AP buffer containing nitroblue tetrazolium and 5-bromo-4-chloro-3-indoyl phosphate and stopped by incubation in 2 X SSC.

Detection of Chromosome Aberrations Using CISS Hybridization

Using the above-described steps, it is possible to specifically stain or label any selected individual chromosome (or chromosomes) referred to as a target chromosome, or a subregion(s) thereof. As explained in the examples, the present method has been shown to be useful in a variety of cells, both in mitotic (e.g., metaphase, prophase) and interphase cells. As described in detail in Example 2, the CISS hybridization method of the present method is useful for rapidly screening mitotic and interphase aneuploid tumor cells for complex numerical and structural aberrations of individual chromosomes (e.g., changes in number of chromosomes, deletions and rearrangements or translocations).

In this context, biotinylated library DNA inserts were used in the CISS hybridization method to produce hybrid molecules which were detected using known techniques. Two glioma lines were used as general models of aneuploid cells, particularly tumor cells. One was an oligodendroglioma line and the other was a gliobastoma line. These were analyzed, using the biotinylated DNA probes specific for chromosome 1, 4, 7, 18 and 22. Specific labeling of the chromosomes, from pter to qter, made it possible to visualize numerical changes, deletions and rearrangements in these chromosomes in metaphase spreads and in early prophase and interphase nuclei. Complete chromosomes, deleted chromosomes and segments of translocated chromosomes were rapidly delineated in the very complex karyotypes of such cells. Additional subregional probes were also used to further define aberrant chromosomes. Digital image analysis was used to quantitate the total complement of specific chromosomal DNAs in individual metaphase and interphase cells of each line. Under-representation of chromosome 21 and over-representation of chromosome 7 (specifically 7p) were observed. This is in agreement with previous observations by others using conventional cytogenetic bauding techniques. Bigner, S.H. et al., Cancer Genet. Cytogenet., 22:121-135 (1987); Shapiro, J.R., Semin. Oncol., 13:4-15 (1986).

The two glioma cell lines used display several cytogenetic features common to many glioma cells. Thus, it is reasonable to expect that the CISS hybridization method can be used in a similar manner to specifically decorate other chromosomes and to detect those chromosomes in glial tumors. The two cell types analyzed are highly aneuploid (i.e., they have 100 chromosomes, rather than the normal 46). Therefore, it is reasonable to expect that the CISS hybridization method can be used in assessing any type of aneuploid (tumor) cell.

Thus, the CISS hybridization method can be used in assessing chromosomal aberrations associated with cancer, both in diagnosis of the disease and in monitoring its status (.e.g., progression, regression or change with treatment) in patients. In this application, assessment of a single chromosome or of multiple chromosomes, and subregions thereof, can be carried out. Double hybridizations using two DNA probes, each bearing a different label can also be carried out. That is, biotinylated chromosome 7 library DNA inserts and a probe specific for alphoid repeats on chromosome 7 (pa7tl) which was modified with aminoacetylfluorene (AAF) were used to assess chromosome 7 content/characteristics in both metaphase spreads and interphase nuclei of the two types of tumor cells (TC 593, TC 620). After hybridization, biotinylated chromosome 7 inserts were detected using avidin-FITC and chromosome 7-specific alphoid AAF labeled sequences were detected with tetramethylrhodamine isothiocyanate (TRITC) conjugated second antibodies. Double CISS hybridization was used to detect translation between chromosome 8 and 14, Burkitt lymphoma cells, a high malignancy form of B lymphocyte tumors such were seen in both metaphase spreads and interphase cells.

This made it possible to detect similarities and differences in chromosome number 7 present in the two tumor cell types: only the four complete number 7 chromosomes found in TC 593 contained a detectable 7 centromeric signal; a smaller and metacentric number 7 chromosome lacked the 7 alphoid sequences and a small block of heterochromatin at 7q11 (indicating that it lacked a characteristic centromeric region). In contrast, all four chromosome number 7 of TC 620 were labeled with the 7 alphoid probe. Double CISS hybridization also made it possible to distinguish among number 7 chromosomes present in one cell type (TC 593) and to demonstrate similarity (at least as to the characteristics assessed) among number 7 chromosomes present in the other cell type (TC 620).

Double CISS hybridization was used to detect translocations between chromosome 8 and chromosome 14 in Burkitt's lymphoma cells; Burkitt's lymphoma is a highly malignant form of B lymphocyte tumors. Translocations were detected in both metaphase spreads and interphase cells.

It is possible, through the use of appropriately-selected probes and/or labels to increase the number of different chromosomes, as well as the number of subregions on some or all of those chromosomes, which can be analyzed simultaneously using multiple CISS hybridization. For example, it is possible to use more than one probe, each specific for a subregion of a target chromosome, to analyze several subregions on that single chromosome at one time. It is also possible to label each probe set (set of DNA or RNA fragments) with a distinct fluorochrome or different reporter molecule, which can be distinguished from one another, after probe-target chromosome hybridization has occurred, by known techniques (e.g., by using specific fluorescent or enzyme reagents).

Furthermore, a "combinatorial" variant of CISS hybridization can be used to enhance the number of chromosomes which can be assessed simultaneously. That is, it is possible to use a hybridization probe mixture made from a single set of probe sequences composed of two halves, each separately labeled with a different fluorochrome (e.g., fluorescein and rhodamine), which, upon hybridization, produce a third fluorescence "color" or signal optically distinguishable from each of the original individual fluorochromes. Pairing of two different fluorochromes in this manner makes it possible to identify three different chromosomes. For example, a probe set labeled only with fluorescein will yield one color upon hybridization; the same probe set labeled only with rhodamine will yield a second (different) color upon hybridization. When half of the probe set is labeled with one of the two, both sequence subsets can hybridize to target with equal probability and be perceived as a third (different) color (in a way not dissimilar to mixing paint). It is important here that two fluorochromes are not introduced into the same molecule, in order to minimize the possibility of E transfer (a well-known process where light emitted by one fluorochrome whose spectrum overlaps that of the other fluorochrome is absorbed by the second fluorochrome. The transferred electrons are emitted by second fluorochrome, which leads to quenching of the first fluorochrome. Pairwise combinations of three different fluorochromes selected for their spectral characteristics can be used singly and in pairwise combinations to produce in a similar manner. This can result in the production of six different fluorescent colors or signals (e.g., three pairs plus three single fluorochromes). Similar combinations of four different fluorochromes results in production of 10 different fluorescent colors or signals, of five different fluorochromes results in production of 15 different colors or signals, etc. This principle of combinatorial fluorescence (combining two or more fluorochromes to label the same probe set) is applicable to metaphase and interphase chromosome analysis because each chromosome is a physically separate entity and is, thus, a distinct target. Composite probe labeling in which mixtures of three different fluorochromes are used provides even greater diversity of colors or signals useful in simultaneous multiparameter analysis.

Another approach to enhance the number of chromosomes which can be analyzed simultaneously involves a "time-reoolved" method of fluorescence detection. In this instance, the DNA (or RNA) probes are labeled with chelating "cages" which bind specific lanthanides (e.g., Europium, turbium). Such metal chelates can be made to fluoresce. They exhibit excited state lifetimes that are much longer (micro to millisec) than those of most normal fluorochromes (whose half lives are in the nanosecond range). Both the wavelength and the fluorescence lifetime is influenced by the nature of the lanthanide metal ion employed. If a pulsed-gating system, which excites the sample with light for a few nanoseconds and then shuts off is used, it is possible to let short-lived fluorochromes decay to their ground state, open the detector system at a defined time after excitation, (i.e., 1-100 microseconds) and detect only long-lived fluorochrome. This method can be used to discriminate 2 fluorescent dyes which have identical spectra but different lifetimes, thus adding a time factor to fluorochrome discrimination.

Another approach to increase the number of different chromosomes that can be analyzed simultaneously is based on a detection system which distinguishes chromosomes in terms of the flexibility or rigidity of an attached fluorochrome. Here, two single stranded probe sets can be labeled with the same fluorochrome, in one probe set the fluorochrome is introduced into the body of DNA sequences which will form hybrid molecules with the target DNA of interest. In the second probe set, the fluorochrome is introduced into DNA sequences, that do not hybridize with the target DNA (e.g., by adding a 3'-tail of poly dA-fluorochrome with deoxynucleotide terminal transferase, ligation of fluorochrome-labeled heterologous DNA to the probe DNA or other conventional secondary labeling techniques known in the art). Fluorochromes within the body of the DNA which form probe-target chromosome hybrids will become immobilized and thus will be unable to rotate freely in solution. In contrast, fluorochromes in the single-strand DNA that is not involved in hybrid formation are not immobilized and can rotate much more freely in solution. By measuring the rate of fluorochrome rotational freedom, (i.e., by measuring how fast the fluorochromes become depolarized when illuminated with polarized light) one can discriminate the two sets of probes.

Use of CISS hybridization and regionally defined probe sets for rapid assessment of chromosome aberrations associated with genetic disorders and chromosomal damage

It has been demonstrated that the CISS hybridization method is useful for the rapid assessment of chromosome aberrations (such as numerical and structural aberrations of chromosome 21) associated with genetic disorders (e.g., in the case of chromosome 21, Down syndrome). DNA probe sets which specifically label the terminal band 21q22.3 or decorate the entire chromosome 21 aberrations in metaphase and interphase cells are described in Example 3. The cloned DNA fragments from the human chromosome 21 are useful to specifically label the cognate chromosomal region in metaphase spreads and interphase nuclei in a variety of cell types. That is, CISS hybridization using a chromosome 21 probe set was shown to be effective in labeling/identifying chromosome 21 DNA in lymphocytes, embryonic chorionic villi cells and a glioma tumor cell line (TC 620). Unique probe sets from band 21q22.3 were also used to detect chromosome 21 in solid tissue ("normal" human brain tissue). Thus, CISS hybridization and hybridization with pools of unique sequence probes clearly have potential as a diagnostic for Down syndrome and for other genetic diseases or other conditions associated with chromosomal aberrations.

Results demonstrate that a trisomic karyotype can be diagnosed easily in interphase cells because the majority of the nuclei (55-65%) exhibit three distinct foci of hybridization. In contrast, less than 0.2% of nuclei in lymphocytes with a disomic karyotype show three nuclear signals; interestingly, the percentage of such nuclei in normal CV cells was higher but still considerably less than 5%. In general, as few as 20-30 cells were sufficient to unambiguously distinguish between disomic and trisomic cell populations. However, in view of the uncertainty of the level of chromosome 21 mosaicism in clinical samples, the number of cells required to make an unambiguous diagnosis will likely be higher. Additional clinical correlations will be required to establish the absolute number. Nevertheless, this analytical approach could allow the diagnosis of Down syndrome without the need to culture cells or to obtain metaphase spreads. It would also decrease the time required to make the diagnosis, from the current 10-14 days to 1 day or less.

Although selected plasmid clones containing only unique human DNA sequences were used here, cosmid clones containing repetitive sequences can also be used to specifically label their cognate genomic region in metaphase and interphase cells by applying hybridization protocols like CISS hybridization that suppress the signal contribution of repetitive sequence elements. Therefore, single or nested sets of cosmids could be used as diagnostic tools for other genetic diseases in a fashion similar to that reported here. Trisomy of chromosomes 13, 18 and 21 and numerical changes in chromosomes X and Y together account for the vast majority of numerical chromosome abnormalities identified during prenatal karyotyping. With the continued development of multiple nonisotopic probe labeling and detection systems it should be possible to visualize three or more chromosomes simultaneously following in situ hybridization. The variations, described in the previous section, of the CISS hybridization method which increase the number of chromosomes, and/or the number of chromosome regions which can be assessed simultaneously can also be used for detecting chromosomal aberrations associated with genetic disorders and chromosomal damage. Thus, the development of a rapid and automated screening test to detect the major trisomic disorders directly in interphase cells from amniotic fluid or chorionic villi cells is a viable future objective. The analysis of specific human chromosomes by in situ hybridization has already been used to complement conventional cytogenetic studies of highly aneuploid tumor lines (Example 2) and the extension to prenatal diagnostic applications seem warranted.

The analysis of karyotypes with translocations of chromosome 21 shows the usefulness of a regional probe set to rapidly identify and characterize even small translocations by unambiguous signals on metaphase chromosomes, thus circumventing an extensive analysis by high-resolution banding. In contrast, the library insert probe is more suitable for defining the relative amount of chromosome 21 DNA that has been translocated. By analyzing interphase nuclei, one can also determine if a balanced or unbalanced number of chromosomal regions exists. However, the detection of a translocated chromosome directly in nuclei would require double-labeling techniques to identify the recipient chromosome to which the chromosome 21 material was translocated. With prior knowledge of the chromosome in question, such translocation events could be assessed by measuring the juxtaposition of the nuclear signals. Rappold, G.A. et al., Hum. Genet., 67:317-325 (1984).

A cosmid clone spanning the entire muscular dystrophy (MD) locus on chromosome X has been used to identify translocation between chromosome X and chromosome 4.

Probes containing 6 kb of sequence were localized in both metaphase spreads and interphase cells with high efficiency. This detection sensitivity with nonisotopic reagents is similar to that achieved in other recent reports. The combination of nonisotopic in situ hybridization with DAPI or BrdUrd banding or total chromosome decoration with library DNA probes thus provides a simple and general approach for gene mapping. Combinatorial fluorescent technology will also make it possible to examine several chromosomal regions simultaneously, thus permitting genetic linkage analysis by in situ hybridization. It also should facilitate the use of small DNA probes to rapidly pinpoint the breakpoints on translocation chromosomes, which could further aid in defining the genomic segments critical for Down syndrome.

Identifying and Isolating Chromosome-Specific Sequences Using CISS Hybridization

The CISS hybridization method of the present invention can also be used to identify chromosome-specific sequences and, subsequently, to separate them from repetitive sequences, using known techniques. Such chromosome-specific sequences, separate from the non-specific or repetitive sequences, and labeled, can be used in hybridization assays carried out, for example, in a diagnostic context, to identify, detect, and/or quantitate a chromosome or chromosome region of interest (e.g., one which is associated with a genetic disorder or causes an infectious disease). Combination of a sample to be assayed for a selected target nucleic acid sequence or sequences and appropriately-selected, labeled chromosome-specific sequences separated from repetitive sequences (e.g., sequences specific for sequences on the chromosome(s), generally referred to as target nucleic acid sequences, which are to be detected and/or quantitated in the sample under appropriate conditions results in hybridization with complementary sequences present in the sample. Hybridization will not occur, of course, if complementary sequences are not present in the sample.

Such separated chromosome-specific nucleic acid sequences can be incorporated into a kit to be used for identification, detection and/or quantitation of chromosomes or chromosome regions of interest, using standard hybridization techniques. For example, labeled nucleic acid sequences which are chromosome 21 specific (or specific to a portion of chromosome 21), identified by CISS hybridization, and separated from repetitive sequences present on chromosome 21, can be included in a kit, along with other reagents such as buffers, competitor DNA, carrier DNA and substances needed for detection of labeled chromosome 21-derived nucleic acid sequences hybridized to chromosome 21 sequences present in a sample. Such kits clearly can be produced to include chromosome-derived nucleic acid sequences from one or more chromosome(s) of interest. Competitor DNA, carrier DNA and substances useful for detecting hybridized sequences will be as described above.

EXAMPLE 1 Cyto-Specific Staining of Individual Human Chromosomes Using Genomic DNA Libraries in CISS Hybridization

DNA libraries

The following human chromosome genomic libraries were obtained from the American Type Culture Collection: LA01NS01 (chromosome 1), LL04NS01 (chromosome 4), LA07Ns01 (chromosome 7), LL08NS02 (chromosome 8), LA13NS03 (chromosome 13), LL14NS01 (chromosome 14), LL19NS01 (chromosome 18), LL20NS01 (chromosome 20), LL21NS02 (chromosome 21), LA22NS03 (chromosome 22), LA0XNL01 (chromosome X). Amplification of these phage libraries on agar plates (using LE 392 cells as the bacterial host), purification of the λ phages and extraction of phage-DNA pools were carried out according to standard protocols. Maniatis, T. et al., Molecular Cloning: A Laboratory Manual, Cold Spring Harbor Laborabory, Cold Spring Harbor, NJ (1982).

Preparation of metaphase spreads and fibroblast cells

Phytohemagglutinin-stimulated lymphocytes from a normal adult male (46, XY) were cultured in McCoy's 5A medium (GIBCO), arrested with Colcemid, treated with a hypotonic solution of 0.075 M KCl, fixed in acetic acid-methanol and metaphase spreads made by standard procedures. Low-passage normal human foreskin fibroblasts (46, XY) were grown on microscope slides, fixed with paraformaldehyde, and permeabilized as described for study of preparations with a more intact three-dimensional structure. Manuelidis, L, Ann. NY Acad. Sci., 450:205-221 (1985).

Preparation of DNAs for in situ hybridization

Insert DNA probes. Genomic DNA fragments from the chromosomal DNA libraries were separated as a pool from the Charon 21A vector arms by digestion with the appropriate restriction enzyme [EcoRI (LA libraries) or Hind III (LL libraries)], followed by preparative electrophoresis in 0.6% agarose gel. The insert fragments were isolated from gel slices by electroelution into an Elutrap (Schleicher and Schuell) and further purified by Elutip-d column chromatography (Schleicher and Schuell). The DNA was then extracted with phenol/chloroform (1:1) and ethanol precipitated. This pool of DNA fragments was labeled either by nick translation using Bio-11-dUTP or by random primer extension with the multiprime DNA labeling system (Amersham) substituting dTTP with 0.5 mM Bio-11-dUTP. Langer, P.R. et al., Proc. Natl. Acad. Sci. USA, 78:6633-6637 (1981) and Brigati, D.J. et al., Virology, 126:32-50 (1983). Alternatively, the DNA of the chromosome-specific libraries was biotin-labeled directly (without separation of the vector arms) by nick translation.

Probe size. To facilitate probe penetration and to optimize reannealing hybridization, labeled DNA fragments smaller than 500 nucleotides are used; the majority of the probes are generally 150 to 250 nucleotides in length. DNAse concentrations were empirically established in nick-translation reactions to yield fragments in the desired size range and this was verified by agarose gel electrophoresis. Random primer extensions were also carried out under conditions which yielded a comparable DNA size distribution.

Competitor DNA. Human genomic DNA (from placenta or white blood cells), prepared as described, as well salmon testis genomic DNA (Sigma) were digested with DNAse to obtain fragments with the same size distribution as the probe DNA, then extracted with phenol/chloroform and ethanol precipitated. Davis, L.G. et al., "Basic methods in molecular biology", Elsevier, New York Amsterdam (1986). These competitor DNAs were used in varying ratios with probe sequences, as described with reference to Figure 2.

Preannealing and hybridization. Under standard conditions, from 5 µg/ml to 30µg/ml of biotin-labeled DNA, representing library insert fragments, and varying amounts of competitor DNAs were combined, ethanol-precipitated and resuspended in formamide. The probe concentration was adjusted to reflect the relative DNA content of each chromosome target. For example, chromosome 1 contains approximately 5.3 times as much DNA as chromosome 21; thus the probe concentrations used were 30 µg/ml and 5 µg/ml, respectively. Mendelsohn, M.L. et al., Science, 179:1126-1129 (1973). When total library DNA was used as the probe mixture instead of purified DNA inserts, 10 times as much labeled DNA was added to compensate for the large amount of vector sequences. In the case of the X-chromosome library, LA0XNL01, only twice as much labeled library DNA was used, since the human DNA inserts constitute almost half of the total DNA. For comparative purposes, the concentration of human competitor DNA in the hybridization mixture was varied from 1 to 1.0 mg/ml and salmon testis DNA was added as required to result in a final DNA concentration of 1.0 mg/ml in 50% formamide, 1 x SSC (0.15 M sodium chloride, 0.015 M sodium citrate, pH 7.0) and 10% dextran sulfate. These solutions were heated at 75°C for 5 min. to denature the DNA and then incubated at 37°C for various times to promote partial reannealing. The preannealing step was done in an Eppendorf tube just prior to applying the hybridization mixture to the specimen. Nuclei and chromosome spreads on glass slides were incubated in 70% formamide, 2 X SSC] at 70°C for 2 min. to denature chromosomal DNA and then dehydrated in a series of ice-cold ethanal (70%, 90% and 100%, each for 3 min.). After application of the preannealed probe mixture (2.5 µl/cm$^2$) to slides pre-warmed to 42°C, a coverslip was added and sealed with rubber cement. The samples were then immediately incubated at 37°C in a moist chamber for 10-20 h.

In those cases where paraformaldehyde fixation was used to more optimally preserve the 3-D structure of the specimen, the slides were equilibrated in 50% formamide, 1 X SSC (2 X 5 min.), excess fluid was removed without permitting the sample to dry, the probe mixture was added (5 µm/cm$^2$), and a coverslip mounted and sealed with rubber cement. Manuelidis, L., Ann. NY Acad. Sci., 450:205-221 (1985). Denaturation of both probe and cellular DNA was done at 75°C for 5 min. before hybridization was allowed to proceed overnight at 37°C.

Detection

After hybridization, the slides were washed in 50% formamide, 2 X SSC (3 X 5 min., 42°C) followed by washes in 0.1 X SSC (3 X 5 min., 60°C). Thereafter the slides were incubated with 3% bovine serum albumin (BSA), 4 x SSC for approximately 30 minutes at 37°C. Detection of the biotinylated probe was achieved using either fluorescein-labeled avidin or avidin-alkaline phosphatase complexes. All detection reagents were made up in 4 X SSC, 0.1% Tween 20, 1% BSA and all washes were carried out in 4 X SSC, 0.1% Tween 20 (3 X 3 min., 42°C). For fluorochrome detection, slides were incubated with 5 µg/ml fluorescein isothiocyanate (FITC)-conjugated avidin DCS (Vector Laboratories) at 37°C for 30 min., followed by washes. In rare cases, the FITC signal was amplified by incubation with 5 µg/ml biotin-conjugated goat anti-avidin D antibodies (Vector Laboratories) at 37°C for 30 min., followed by washing, a second incubation with 5 µg/ml FITC-conjugated avidin (37°C, 30 min.) and a final wash. Pinkel, D. et al., Proc. Natl. Acad. Sci. USA, 83:2934-2938 (1986). For detection by enzyme activity, samples were incubated with 2.5 µg/ml streptavidin, washed, incubated with 2 µg/ml biotin-conjugated alkaline phosphatase (Vector Laboratories), washed again and pre-equilibrated in Ap-buffer 9.5 (100 mM Tris-HCl, pH 9.5, 100 mM NaCl, 50 mM MgCl$_2$) for 2 x 5 min. at room temperature. The enzyme reaction was carried out in AP buffer 9.5 containing 330 µg/ml of nitroblue tetrazolium (NBT) and 165 µg/ml 5-bromo-4-chloro-3-indolyl phosphate (BCIP) at 37°C for 0.5-1 hour and stopped by incubation in 2 X SSC. All preparations were counterstained with 200 ng/ml 4,6-diamidino-2-phenylindoledihydrochloride (DAPI), 2 X SSC for 5 min. at room temperature and mounted in 20 mM Tris-HCl, pH 8.0, 90% glycerol containig 2.3% of the DAPCO antifade, 1,4 diazabicyclo-2(2,2,2)octane. Johnson G.D. et al., J. Immunol. Methods, 55:231-242 (1982).

Densitometry

A graphics workstation (VAX station II/GPX, Digital Equipment Corporation) with a frame grabber (ITEX FG-101, Imaging Technology) and a Dage-MTI-65 video camera with a Zeiss S-Planar 60 mm lens were used as described in Manuelidis, L. and J. Borden, Chromosoma, 96:397-610 (1988). Images were digitized directly from the negatives and stored on disk. Background was removed and polygonal regions around each chromosome were defined. Threshold density levels were used to outline chromosome regions within the defined polygonal areas. Means density levels within these outlined chromosome regions, R, were determined by the total signal $\int I(x,Y)dR/area$ R, where $\int I(x,y)$ is the pixel intensity (0-225) at each point within the region R. The threshold background intensity was subtracted from the mean regional density, both for labeled chromosome 7 and for background chromosomes. The signal to noise ratio was calculated as mean chromosome 7 signal/mean background chromosome signal.

The following is a description of the results of the work described above, which clearly demonstrate specific labeling of the individual chromosome indicated. The first sections describe use of chromosome library inserts labeled with biotin and the second describes use of DNA insert fragments.

Figure 2 shows suppression of signals from cross-reacting sequences within a chromosome 7-derived DNA library by different concentrations, as described below.

Figure 2A shows chromosome 7 library inserts labeled with biotin and hybridized to metaphase spreads from normal human lymphocytes without human competitor DNA. Prominent labeling of the two no. 7 chromosomes is observed; additionally, a distinct band-like patterns of hybridization is seen on most of the other chromosomes, and two E-group chromosomes are especially brightly stained. This general chromosomal banding pattern resembles R-banding, and suggests that a significant portion of the background cross-hybridization signal originates from Alu repetitive sequences. Previous studies have shown that Alu sequences delineate an R-banding pattern, while Giemsa positive-banding profiles are highlighted by KpnI interspersed repeats. Manuelidis, L. and C.D. Ward, Chromosoma, 91:28-38 (1984).

Establishment of experimental procedure to eliminate the hybridization signal from repetitive elements

A series of pilot studies were therefore undertaken to establish experimental parameters to eliminate the hybridization signal from such repetitive elements. The kinetics of nucleic acid reassociation in solution are dependent on the total concentration of nucleic acid (Co, in moles of nucleotides per liter) and the time of renaturation (t, in seconds). When reassociation conditions are standardized for temperature (taking into account the formamide concentration), cation concentration and buffer system, the reassociation kinetics are comparable with respect to Cot values. Under defined conditions, the fast reassociating fraction of mammalian genomes containing the highly repetitive DNA is completely reannealed at Cot values between $1 \times 10^{-1}$ and $5 \times 10^{-1}$; the intermediate fraction containing the middle repetitive DNA is completely renatured at a Cot value of $1 \times 10^{2}$. Britten, R.J. and D.E. Kohne, Science, 161:529-540 (1968). Thus at a human DNA concentration of 1.0 mg/ml (corresponding to $3 \times 10^{-3}$ moles of nucleotide per liter), the fast fraction would be renatured in approximately 10s, whereas the middle repetitive DNA would need more than 9 h to reach complete reannealing. Since the fast fraction of reassociating DNA containing most or all of the ubiquitous repetitive DNA causing cross-hybridization signals, a total DNA concentration of 1.0 mg/ml was used and partial reannealing of the probe mixture was allowed prior to application to specimens. The optimal renaturation time was determined empirically (see below). This was important because the in situ hybridization conditions deviate from the standard conditions under which reassociation kinetics are determined (e.g., hybridization in 50% formamide at 37°C corresponds to 0% formamide at about 70°C; dextran sulfate also increases the reassociation time significantly). Furthermore, it was unclear to what degree the middle repetitive DNA contributed to the non-specific signal and therefore should also be prevented from hybridization by a preannealing procedure.

The stringency for the reannealing and in situ hybridization experiments was determined in 50% formamide at 37°C (adapted from standard in situ hybridization protocols) and 1 X SSC [this cation concentration of 0.165 M comes close to the concentration used in kinetics the study of Britten and Kohne. Britten, R.J. and D.E. Kohne, Science, 161:529-540 (1968)]. Competitor human DNA was added in the reassociation procedure to obtain the desired final high DNA concentration and to maintain a high level of repetition of the DNA sequences that should preanneal. While total human genomic DNA represents all the highly repetitive DNA to be removed by pre-annealing, it also contains sequences of the target chromosome. Thus, the addition of excessive amounts of human DNA would be expected to diminish the chromosome-specific signal. Therefore, the optimal concentration of total human DNA to use as the competitor was first determined. To keep the total DNA concentration constant at 1.0 mg/ml, genomic salmon DNA was added as needed. Salmon DNA shares certain repetitive DNA elements, such as poly dCdA in common with human DNA, but lacks others, most notably the Alu- and KpnI repeats. Hamada, H. et al., Proc. Natl. Acad. Sci. USA, 79:6465-6469 (1982). This results in a lower frequency of the latter sequences with increasing amounts of salmon DNA in the reassociation reaction.

Figure 2 shows typical experimental results obtained when 20 µg/ml of the chromosome 7 probe set was denatured together with 50 µg/ml (B), 100 µg/ml (C), 200 µg/ml (D) or 1000 µg/ml (E) of DNAse-digested human genomic DNA which was preannealed for 20 min. Hybridization and detection using avidin-FITC were carried out as described above. From each preparation ten black and white pictures were taken under standardized photographic conditions for densitometric studies (see below). In the absence of human genomic competitor (A) the signal showed little chromosomal specificity. However, with 50 and 100 µg/ml of human competitor DNA, as increase of label specificity is readily apparent (Figure 2B,C). Specific staining of chromosome 7 was achieved with a peak of signal intensity using 100 and 200 µg/ml of human competitor DNA (Figure 2C, D). Higher concentrations of human DNA caused an apparent decrease of signal intensity, especially at 1000 µg/ml human DNA (Figures 2E). However, the signal obtained under these latter conditions is still reasonably bright to the observer, but requires a different exposure for optimal illustration (not shown).

A computer-assisted method of quantitative densitometry (see above) was used to establish the overall level of labeling specificity. The ratio of fluorescence signal from the target chromosomes of interest to the background fluorescence noise emanating from non-target chromosomes was determined from images digitized from multiple photographic negatives of a DNA titration experiment, as illustrated in Figure 2. The signal-to-noise ratio obtained with each concentration of human competitor DNA is given in Table 1.

Table 1

| Densitometric analysis of the suppression of cross hybridization signals by concentrations of human competitor DNA | | | | | |
|---|---|---|---|---|---|
| DNA conc. µg/ml) Human competitor DNA conc. | Signal | | Noise | | Signal-to-noise ratio Confidence interval[c] (99%) |
| | Pixel[a] | η | Pixel[b] | η | |
| 0 | 71.48 | 8 | 54.66 | 26 | 1.31±0.04 |
| 50 | 74.50 | 8 | 37.43 | 28 | 1.99±0.07 |
| 100 | 162.64 | 8 | 20.06 | 23 | 8.11±0.35 |
| 200 | 147.35 | 8 | 20.53 | 26 | 7.18±0.37 |
| 500 | 89.78 | 8 | 18.63 | 21 | 4.82±0.28 |
| 1000 | 94.37 | 8 | 30.51 | 17 | 3.09±0.12 |

[a] Mean value of pixel intensity of target chromosome

[b] Mean value of pixel intensity of non-target chromosomes (from the same metaphase spreads)

[c] The confidence interval was calculated using Fieller's theorem (Finney, D.J., Statistical methods in biological assay, 2nd edn., Hafner Press, N.Y., 1971)

η Number of chromosomes from which the mean was determined

**EP 0 444 115 B2**

Optimal reannealing conditions for suppression of nonspecific signal (using 20 μg/ml of chromosome 7 probe and 100-200 μg/ml human genomic (DNA), gave a signal-to-noise ratio of ca. 8:1. Additional attempts to improve the signal to noise ratio by increasing hybridization stringencies (e.g., 60% formamide or 0.2 x SSC) gave no apparent improvement and led to an overall decrease in signal intensity.

Since about 100-200 μg/ml of human competitor DNA was shown to give the optimal specificity, 200 μg/ml was used for another analysis of signal specificity with respect to the renaturation time (see above). After 0, 2, 5, 10, 20, 40 and 50 min. of preannealing, aliquots were taken and used for in situ hybridization experiments as before. As indicated in Figure 3, specific labeling was obtained for all preannealing times. A small improvement of the signal is seen with increasing renaturation times from 0 to 20 min. Longer renaturation times up to 60 min. (not shown) gave no significant improvement in signal strength or chromosome specificity. The subjective impression of a signal improvement with 20 min. of preannealing (Figure 3D) could not be confirmed by a densitometric analysis, carried out as described above, since no significant differences in the signal-to-noise ratio of the different preannealing times were observed (data not shown). Therefore, the standard renaturation time in all subsequent experiments was 10-20 min. Since a signal is clearly visible at renaturation time 0, the few seconds necessary for transferring the probe mixture to the microscope slide appear to be sufficient to effectively preanneal many of the sequences that cause nonspecific labeling by cross-hybridization. Furthermore, the large excess of single-stranded competitor DNA may efficiently compete with the biotinylated probe sequences for ubiquitious chromosomal target sites during the hybridization reactions. These results demonstrate that the majority of highly repetitive DNA sequences can be sufficiently suppressed to achieve chromosome-specific labeling by in situ hybridization.

In certain cases the signal distribution over the entire chromosome shows some variability from experiment to experiment. When the overall signal is decreased, some chromosomal subregions show a brighter staining; these signal hotspots generally constitute chromosomal sites that contain known chromosome-specific repetitive sequences. In the experiments shown in Figures 2 and 3, predominent staining of the centrometric region of chromosome 7 is seen, which corresponds to the chromosome-specific signal of an alphoid repetitive DNA. Waye, J.S. et al., Mol. Cell Biol., 7:349-356 (1987) and see Example 2. Apparently, the abundance of these repeated sequences is sufficiently low to prevent their suppression under the conditions used here. The unequal signal distribution can be overcome by amplifying the overall signal using an antibody sandwich technique as described above. Furthermore, a predominant staining of the region 1q12 that corresponds to the chromosomal site of satellite III DNA was frequently observed in labeling chromosome 1. Cooke, H.J. and J. Hindley, Nucleic Acids Res., 6:3177-3197 (1979) and Gosden, J.R. et al., Cytogenet. Cell Genet., 29:32-39 (1981) and see Example 2. An example of the balanced signal distribution seen after such an amplification step is shown in Figure 4A.

Several commercially available DNA libraries, each representing a single human chromosome, were tested for their ability to specifically label the chromosome they represented, under the standardized reannealing conditions described above and with the probe concentrations adjusted for chromosome size, as described above. Some examples, for chromosomes 1, 4, 7, 13, 18 and 20, as shown in Figure 4, clearly demonstrate that specific labeling can be achieved with most chromosome libraries. Table 2 lists the libraries tested with their relative scores of labeling specificity. All scores are positive because the chromosome of interest was always decorated. The highest score (4+) is used when no significant cross-hybridization to other chromosomes was observed and the scores decrease (3+ to 1+) with an increasing amount of cross-hybridizing sequences.

Table 2

| Relative quality of specific chromosome labeling in situ using preannealed biotinylated library DNA inserts | | |
| --- | --- | --- |
| Chromosome | Library used (ATCC designation) | Relative specificity of in situ hybridization signal[a] |
| 1 | LA01NS01 | 3+ |
| 4 | LL04NS01 | 4+ |
| 7 | LA07NS01 | 4+ |
| 8 | LL08NS02 | 4+ |
| 13 | LA13NS03 | 1+ |
| 14 | LL14NS01 | 2+ |
| 18 | LL18NS01 | 4+ |
| 20 | LL20NS01 | 4+ |
| 21 | LL21NS02 | 3+ |
| 22 | LA22NS03 | 3+[b] |
| X | LA0XNL01 | 4+ |

[a] See the text for score definition

[b] Under standard preannealing conditions the chromosome 22 library gave a score of +1; a value of +3 was achieved only with a human competitor DNA concentration $\geq$700 $\mu$g/ml (total DNA concentration 1.0 mg/ml).

All attempts to reduce the additional signals on other chromosomes by varying the experimental conditions failed except in experiments with chromosome 22; in this case higher concentrations of human competitor DNA (700 $\mu$g/ml) resulted in a significant improvement of signal specificity. The library exhibiting the lowest chromosome specificity was the chromosome 13 library (Figure 4E). Multiple minor binding sites on other chromosomes, as well as an exceptionally bright staining of Yq12 were observed; the signal on the Y chromosome was visible using either female or male human DNA as the competitor. None of the experimental parameters tested improved on the overall specificity of this library.

Remarkably, a weak signal or even absence of signal can be observed at the centromeric region of some chromosomes (see chromosomes 4 and 18, Figure 4C,D). In contrast to chromosomes 1 and 7, which contain chromosome-specific repetitive elements, the centromere regions of chromosomes 4 and 18 apparently contain repetitive sequences, most likely alphoid satellite DNAs, which are very abundant and thus are suppressed by the reannealing technique. However, these chromosomal regions are very small and the effect can only be observed when the corresponding chromosomes are fairly elongated.

Biotinylated total library DNA (containing the phage vector sequences) was also used as probes, in concentrations adjusted to the amount of human DNA inserts. (see above). One example is shown in Figure 4F with the chromosome 20 library. Although good staining of the chromosome of interest generally was achieved, significant nonspecific background on the entire slide was a common problem. Similar results were obtained with plasmid libraries containing human DNA subcloned from the lambda phage libraries. In contrast, there was no background problem with the total chromosome library LA0XNL01, which contains a significantly smaller proportion of vector sequences in the probe mixture since the size of the human DNA inserts is much larger.

The suppression of repetitive sequences by this reannealing technique also permits the use of flow-sorted chromosome libraries to detect chromosomal domains within interphase nuclei. Typical examples of results obtained after hybridization of chromosome 1, chromosome 7 and chromosome 18 probe sets to normal human lymphocytes after acetic acid-methanol fixation are shown in Figure 5. Discrete focal domains of hybridization signal are seen with all libraries that had scores of 2+ or more (see Table 2).

Most nuclei (n $\geq$ 100 per estimate) exhibited two domains (60%-70%); however, a significant number showed only a single domain (20%-30%) or no hybridization signal at all (5%-10%). Accordingly, ca. 95% of male nuclei exhibited one and ca. 5% showed no hybridization signal when the X chromosome library DNA was used as probe. Notably, no nuclei with three domains were found with any of the chromosomal probe sets tested. In contrast, all metaphase spreads showed the decoration of both chromosome homologs without exception. This interphase variability may reflect, in part, the close juxtaposition of two individual domains in some cells, or the inability to resolve domains that actually occupy different areas within the nuclear volume but are unresolved when examined by two-dimensional imaging methods (see Fig. 5D; for discussion see also Cremer et al., Exp. Cell Res., 176:199-220 (1988). The small number of nuclei exhibiting no hybridization signal may be a reflection of suboptimal hybridization conditions. It is of interest to note that the size of the intranuclear domains correlates reasonably well with the relative size of the cognate metaphase chromosome. These observations provide a definitive proof that the DNA of individual chromosomes exhibits a clear territorial organization in the interphase nucleus of a normal human cell.

Acetic acid-methanol fixed nuclear spreads, such as those shown in Fig. 5, clearly retain the territorial organization for each of the chromosomes examined; however, the nuclear structure is not optimally preserved. Additional studies with specimens that possess better preservation of 3-D structure using paraformaldehyde fixed human diploid fibroblasts and a laser-scanning confocal fluorescence microscope assembly for 3-D image reconstruction have been done. The cells were fixed and permeabilized as described by Manuelidis and hybridized with chromosome library probes as outlined above. Manuelidis, L., Ann. NY Acad. Sci., 450:205-221 (1985). The probe-competitor DNA mixture was applied directly to the slide and denatured at the same time as the cellular DNA. Results showed the arrangement of the chromosome 7 domains in the nucleus and the frequently observed helical structure of labeled chromatic within chromosome domains. The degree to which this helicity reflects true domain substructure or is an artifact reflecting preparation and fixation procedures is currently being investigated. Nevertheless, this preliminary observations establishes the feasibility of using chromosome specific probes to analyze the topography of chromosomal domains in the interphase cells.

EXAMPLE 2 Detection of Chromosome Aberrations in Tumor Cells by CISS Hybridization Using Chromosome-Specific Library Probes

Cells

TC 593 is a pseudotetraploid cell line (modal chromosome number, 83) established from a human glioblastoma; it grows in a flat, spreading fashion and contains many process. TC 620 is pseudotriploid with a modal chromosome number of 64 and was established from a human oligodendroglioma; it grows in an epithelial fashion. Both cell lines have been described in detail. Manuelidis, L. and E.E. Manuelidis, In: Progress in Neuropathology, Vol. 4, 235-266, Raven Press, N.Y. (1979). The present experiments made use of subclones C2B (TC 593) and C2B (TC 620) at approximately 180 passages after repeated subcloning from a single cell of the original tumor line cultured as previously described by Manuelidis and Manuelidis (see reference above). Standard hypotonic treatment and acid/methanol fixation of the cells were employed. Cremer et al., Exp. Cell. Res., 176:199-220 (1988).

DNA Probes and Libraries

Phage DNA libraries from sorted human chromosomes were obtained from the American Type Culture Collection: LA01NS01 (chromosome 1), LL04NS01 (chromosome 4), LA07NSO1 (chromosome 7), LL18NSO1 (chromosome 18) and LA22NS03 (chromosome 22). Amplification of these libraries, isolation of human DNA inserts and biotinylation were carried out as described in Example 1. A probe specific for alphoid repeats on chromosome 7 (pa7tl) was the gift of H. Willard and specifically decorates pericentromeric heterochromatin of chromosome 7 under high stringency conditions (60% formamide). Waye et al., Mol. Cell. Biol., 7:349-356 (1987); Cremer et al. Exp. Cell. Res., 176:199-220 (1988). Some DNA probes were modified with aminoacetylfluorene (AAF); and detected as described by Cremer et al. for double labeling experiments. Landegent et al., Exp. Cell Res., 153:61-72 (1984); Cremer, R. et al., Exp. Cell Res., 176:199-220 (1988).

In Situ Hybridization and Detection of Hybridized Probes

CISS hybridization with biotinylated library DNA inserts and detection of hybrid molecules was generally carried out using standard conditions, as described in detail in Example 1. In double CISS hybridizations using biotinylated chromosome 7 library DNA inserts and the AAF-modified 7 alphoid probe, the latter probe was heat denatured separately and only added to the hybridization mixture at the end of the reannealing step at a final concentration of 10 μg/ml (see Example 1).

Digital Image Analysis of Specifically Decorated Metaphase and Interphase Chromosome

A VAX station II/GPX graphics workstation (Digital Equipment Corporation) with an ITEX FG 100-Q frame grabber (Imaging Technology) were used as previously described together with a Zeiss S-Planar 60 mm lens and a Dage-MTI 65 video camera. Manuelidis, L. and J. Borden, Chromosoma, 96:397-410 (1988). Images were digitized from negatives of metaphase spreads and interphase nuclei; the background was removed and polygonal regions were defined to specifically decorated metaphase chromosomes or interphase domains (see Example 1). A scan line algorithm was used to calculate histograms within the polygonal regions. Since the value of the histograms $H(i)$ of a particular intensity (range 0-255) within the defined regions is the number of pixels at that intensity $i$, the area within the region falling within an intensity range $i_0-i_1$ is the integral of the histogram from $i_0-i_1$. Similarly, the 2-D integral in the region defined by the intensity range $i_0-i_1$ equals $\Sigma H(i).i.i_0$ was chosen for each hybridization, in order to properly outline the decorated chromatin and distinguish this area from background regions. $i_1$ was set to the maximum value 255 in order to capture the entire intensity range above the threshold.

Measurements of total signal intensity versus area were designed as a control for the potential presence of variable chromosome domain extension within interphase nuclei. In interphase, a more extended chromosome domain might be expected to have a greater area (or volume) yet a lower fluorescence signal intensity per unit area. If a constant amount of hybridized DNA corresponds to a constant total fluorescence, the total signal intensity is a measure of labeled DNA content. It is also possible to measure 3-D hybridized volumes within nuclei and 3-D integrated total hybridized signals. Manuelidis, L. and J. Borden, Chromosoma, 96:397-410 (1988). The background, b, was subtracted from the discrete 2-D integral $\iint I(x,y)dA$ within a labeled region R, to yield the total signal:

$$Sig_t = \iint I(x,y)dA - b\iint dA,$$

where $dA$ is a single pixel. Similarily, the mean intensity within the region is calculated as 2-D integral/area or $\iint I(x,y)dA/\iint dA$.

The following is a description of the results, with reference to the appropriate figures, of the work described above. They clearly document structural and quantitative changes in the human glioma lines, including loss and gain of entire individual chromosomes and of chromosomal subregions. They also show that it has been possible to characterize both minor and predominant karyotypic features in each cell line. All chromosomes tested to date (i.e., 1, 4, 7, 18 and 22) clearly highlighted numerical and/or structural aberrations, some of which were subtle.

Detection of numerical and structural chromosome aberrations in metaphase spreads.

Figures 6-8 and 12 show typical metaphase spreads from the malignant glioma cell lines TC 620 and 593 after CISS hybridization with biotinylated DNA inserts from each of the human chromosomes 1, 4, 7, 18 and 22. Hybridized inserts were detected with avidin fluorescein isothiocyanate conjugates (FITC) and cells were counterstained with 4,6-diamidino-2-phenylindole dihydrochloride (DAPI). Chromosomes designated as "complete" had an apparently normal size, centromere index and DAPI staining pattern. Despite this designation, these complete chromosomes may contain fine structural aberrations only detectable by additional investigations (see below). Apparently complete chromosomes 1, 4, 7, 18 and 22 were observed in both TC 620 and TC 593. Additionally, other homologs of these chromosomes showed significant rearrangements and abnormalities, including translocations and deletions. The predominant numerical and structural aberrations delineated in each of these cell lines are described below. A minimum of 25 good metaphase spreads were evaluated for each glioma line and for each chromosome. These data are summarized in Fig. 9.

Chromosome 1

In TC 620, the oligodendroglioma line, chromosome 1 inserts decorated two apparently complete 1 chromosomes and two marker translocation chromosomes (Figs. 6A, 6B, 9). One marker was metacentric and contained an entirely decorated 1q arm, but its p arm was from another chromosome (of unknown origin). The other marker chromosome was submetacentric and showed a small segment from another chromosome attached to the 1p arm. In both marker chromosomes breakpoints were localized close to the centromere in 1p11 or 1q11. The identification of the 1p segment was established by DAPI banding (Fig. 6B), by 5'-bromo-2'-deoxyuridine (BrdU) banding, and by hybridization with a 1p36.3 probe (data not shown); the 1p36.3 probe additionally revealed deletion of this subregion in one of the apparently complete 1 chromosomes. The overall picture was of a nearly trisomic representation of chromosome 1, with a common breakpoint, and subsequent translocation.

In TC 593, the glioblastoma line, an even more complex pattern of numerical and structural chromosome 1 aberrations was observed. In a sample of 50 metaphase spreads, the majority (52%) showed six aberrant chromosomes that were decorated; 14% of the metaphases showed five aberrant chromosomes, and 34% showed higher numbers of chromosomes 1 segments (up to 14). Figures 6C, D and 9 show the most typical, predominant karyotype and demonstrates the rapid definition of chromosome 1 abnormalities in this cell line. Aberrations included three acrocentric chromosomes with a consistent breakpoint in 1p1, chromosomes with a deletion of the distal park of 1q, a submetacentric translocation chromosome with a loss of the complete 1q, and an iso(1p) marker chromosome (see Fig. 9).

Chromosome 4

In TC 620, chromosome 4-specific inserts decorated one apparently complete chromosome 4, and three additional chromosomes with segments containing chromosomes 4 DNA (Figs. 7F, 9). These latter segments on translocation chromosomes would have been difficult to rapidly and unambiguously define with banding procedures alone. The smallest of the translocated chromosome 4 segments formed part of an approximately metacentric chromosome. The two larger segments were found on submetacentric chromosomes of different overall size. In the smaller chromosome, the short arm and part of the long arm of 4 were present with an apparent breakpoint at 4q2, i.e., 4pter-4q2. In the larger submetacentric chromosome, a region that may represent the rest of 4 (4q2-qter) appears. Thus the predominant karyotype of TC 620 showed only slightly more than two equivalents of chromosomes 4 (see also the area measurements described below). The non-4 regions have not been further defined.

In TC 593, there were generally only two chromosomes decorated by chromosomes 4 DNA inserts, and both of these were compatible with normal 4 chromosomes. Approximately 30% of the metaphase spreads in TC 593 showed an additional submetacentric chromosome with chromosome 4 material (Fig. 7E). Thus, although both 4 chromosomes were apparently normal, there was a significant under-representation of this chromosome in this pseudotetraploid line (Fig. 9).

Chromosome 7

Three complete 7 chromosomes, and one smaller metacentric chromosome containing translocated 7 material were typically found in TC 620 metaphase spreads (Figs. 8A, 9). The translocated chromosome 7 material included the short arm of chromosome 7 (as shown by DAPI banding; cf. Fig. 8B) and the pericentromeric heterochromatin with the breakpoint in 7q1 (see also below).

In TC 593, five chromosomes entirely decorated by chromosome 7 insert probes were regularly observed (Figs. 7G, 8E). Four of these appeared to represent complete number 7 chromosomes, whereas one was smaller and metacentric. DAPI banding (Fig. 8E, insert) and size measurement (cf. Fig. 13) were consistent with an iso(7p). This conclusion was further supported by double in situ hybridization experiments with biotinylated chromosome 7 inserts (detected with avidin FITC) and chromosome 7-specific alphoid AAF labeled sequences (detected with tetramethylrhodamine isothiocyanate (TRITC) conjugated second antibodies). They showed that only the four complete 7 chromosomes, contained a detectable 7 centromeric signal (metaphase, Fig. 7G,H; interphase, Fig. 7I, J). Thus, the iso(7p) marker chromosome did not have a characteristic centromeric region as it lacked both the 7 alphoid sequences and a small block of heterochromatin at 7q11 (see Fig. 8E, insert). In contrast, all four 7 chromosomes of TC 620 were labeled with the 7 alphoid probe (data not shown).

Chromosome 18

In TC 620, two apparently complete 18 chromosomes and a truncated minute chromosome were entirely decorated (Figs. 8C, D, 9). This truncated chromosome is 18q- (and possibly also 18p-). The rest of the chromosome 18 region(s) was never detected.

Three translocation chromosomes involving chromosome 18 material were typically detected, in addition to an apparently normal chromosome 18 in TC 593 metaphase spreads (Figs. 8F, G, 9). In a minor proportion of metaphases there was a small additional translocation observed. The exact chromosomal region from which this translocated 18 material derived could not be resolved by DAPI staining. The predominant karyotype for 18 is therefore close to tetrasomic in this cell line, but is under-represented in the pseudotriploid TC 620.

Both the 18q-marker chromosome in TC 620 and the three translocated 18 chromosomes in TC 593 hybridized strongly to a chromosome 18-specific alphoid repeat. Accordingly, both intact and aberrant 18 chromosomes could also be counted after in situ hybridization with this centromeric probe. Cremer, T. et al., Exp. Cell Res., 176:199-200 (1988) (see also below). DAPI banding and hybridization to 18-specific alphoid repeats indicated that these translocation chromosomes include the entire 18q region and the centromere, with breakpoints in 18p.

Chromosome 22

Two apparently normal 22 chromosomes were visualized in most TC 620 and TC 593 metaphase spreads (Fig. 10E). It was difficult to ascertain small translocations of this chromosome since hybridization with chromosome 22 inserts resulted in some cross-hybridization to other chromosomes. Some of this cross-hybridization is probably due to shared sequences from the nucleolus organizer regions (on five normal acrocentric human chromosomes) and to shared sequence motifs at the centromeres. McDermid, H.E. et al., Chromosoma, 94:228-234 (1986) and see Example 1. Finally, it should be noted that in constrast to conventional banding analysis, the current experimental approach clearly delineates numerical and structural chromosome aberrations in metaphase spreads of very poor quality (Fig. 10G, H) or in early prophase nuclei (Fig. 10A). These preparations are not accessible to banding analysis, as the chromosomes extensively overlie each other.

Evaluation of Chromosome Domains in Interphase Nuclei

One potential advantage of in situ methods is that individual human chromosomes may be directly visualized as discrete territories in interphase nuclei and thus can be of value in the analysis of solid tumor speciment. Manuelidis, L., Hum. Genet., 71:288-293 (1985); Schardin, M. et al., Hum. Genet., 71:281-287 (1985); Pinkel, D. et al., Proc. Natl. Acad. Sci. USA, 83:2934-2938 (1986). This feature of nuclear topography, also apparent in the malignant cells examined here (Figs. 7A-D, I, 10B-F), was evaluated for its accuracy and diagnostic usefulness. Figure 10A shows three apparently complete 7 chromosomes and one translocated 7p arm in a prophase TC 620 nucleus. Figures 3, and 10B show five chromosome 7 domains in interphase nuclei of TC 593, as previously depicted in metaphase spreads. Figure 10C shows a TC 620 interphase nucleus with two 18 domains of comparable sizes to those seen in normal diploid nuclei (see Example 1). A third, appreciably smaller, decorated 18 domain was also detected and represents the truncated 18 chromosomes seen in metaphase spreads described above. Figure 10D shows four chromosome 18 domains in an interphase nucleus of TC 593, which again is comparable to the numbers in metaphase nuclei. Figure 10E shows a TC 620 interphase nucleus with four chromosomes 1 domains, while Figure 10F shows a TC 593 nucleus with at least five separate chromosome 1 domains (compare Fig. 6A, B and C,D, respectively).

While the hybridization patterns of nuclei shown in Figure 10 were highly characteristic for each cell line, counts of interphase chromosome domains have some inherent difficulties. As an example, Fig. 11A (dark columns) presents an analysis of the counts of labeled interphase domains in randomly selected nuclei of diploid human lymphocytes hybridized with 7 library inserts. Although the number and relative size of chromosome specific domains can be accurately assessed in the majority of nuclei, not all nuclei present a reliable index of the chromosomal constitution, since a considerable fraction of nuclei reveal only one decorated domain and occasional nuclei show no signals. Furthermore all domains are not always cleared separable in these 2-D preparations.

Figure 11 shows representative counts of these preparations. In agreement with TC 593 metaphase counts of chromosome 4, nuclear counts generally showed two clearly separated domains (Fig. 11E). However, the percentage of two-signal preparations was smaller in interphase than in metaphase (45.3% vs. 64%). This artifactual decrease was largely due to a corresponding increased percentage of nuclei showing only one decorated domain or no signal at all. Counts of zero or one domain were not present in metaphase spreads. Significantly, 19.3% of the interphase TC 593 nuclei displayed three clearly separated chromosome 4 domains, and these extra domains were not present in interphase nuclei of diploid human lymphocytes hybridized to this or other libraries under the same conditions (Fig. 10A; Example 1). Finally, the ratio of two versus three domains was identical for both metaphase and interphase cells. Thus interphase nuclei can be reliably used for the detection of extra copies of a single chromosome or chromosomal segment but have limited reliability for detecting the loss of chromosome copies.

In situ hybridization of probes from subregions of interphase chromosomes may more accurately reflect general counts of chromosomal constitution than library probes (Fig. 11A), provided they are done under appropriately high stringency conditions Rappold, G. et al., Hum. Genet., 67:317-325 (1984); Cremer, T. et al., Hum. Genet., 74:346-352 (1986); Cremer, T. et al., Exp. Cell Res., 176:199-220 (1988). However, such regional segment probes do not delineate translocated elements or aberrant chromosomes that lack this segment. Therefore such probes are also not entirely accurate. For example, counts of chromosomes 1 in TC 620 and TC 593 with a probe specific for 1q12 indicated fewer 1 chromosomes than shown here with CISS hybridization (Fig. 9). Cremer, T. et al., Exp. Cell Res., 176:199-220 (1988). Counts of chromosome 7 using only a centromeric sequence further emphasize this point (see above). Double in situ hybridization with the AAF-modified 7 alphoid probe and biotinylated chromosome 7 library inserts typically showed interphase nuclei with five domains, of which only four were simultaneously labeled by the centromeric probe (Figs. 7I, J, 11C). In TC 620, however, both probes gave identical results (Fig. 11B).

Over Representation and Under Representation of Specific Chromosomes

The relative chromosomal dosage in these glioma lines, was also assessed with particular interest in chromosome 7, which has been noted to be generally over-represented in gliomas. Bigner, S.H. et al., Cancer Genet. Cytogenet. 29:165-170 (1986); Shapiro, J.R., Semin. Oncol., 13:4-15 (1986). For comparison, other individual chromosome probes were used as controls. Metaphase chromosomes counts have shown that TC 620 is pseudotriploid with a modal number of 64 chromosomes, while TC 593 is pseudotetraploid with a modal number of 83. Manuelidis, L. and Manuelidis, E.E., IN: Progess in Neuropathology, Vol. 4, pp 235-266, Raven Press, N.Y. (1979). Accordingly, a chromosome and its segments together would be present in a balanced state if three complete copies were present in TC 620, and four in TC 593.

A relative over-representation is present if more than these respective copy numbers can be demonstrated. A number lower than the expected (trisomic or tetrasomic) value indicates that the chromosome is relatively under-represented in the karyotype. In cases where additional DAPI banding information was sufficient to define the selectively decorated abnormal chromosome, the chromosome pieces labeled by the chromosome-specific inserts were put together for analysis (Fig. 9). In the second approach, computer analyses were used to independently verify these results (see below).

TC 620 analyzed by banding showed the equivalent of three 1 chromosome and thus indicated a balanced state for this chromosome. The same was true for the 1p arm in TC 593 which was present in four copies. However, the distal part of 1q was under-represented in TC 593 (see the detailed description given above). In both glioma lines, 7q appeared to be balanced, while 7p was over-represented once in TC 620 and twice in TC 593. Additionally, in both glioma lines chromosomes 22 was clearly under-represented. In order to confirm this finding, double in situ hybridization with inserts of chromosomes 7 and 22 was performed. An example of this is shown in Fig. 12 and demonstrates over-representation of 7 DNA and under-representation of 22 DNA in the same cell. Metaphase counts done in both cell lines by this method of analysis are depicted for chromosome 7 in Fig. 11B, C (dark column) and for chromosome 22 in Fig. 11E. In summary, these two gliomas both show relative under-representation of chromosome 22 and over-representation of the 7p arm. The significant under-representation of chromosome 4 in TC 593, and a portion of 4 in TC 620 is also notable.

Digitized images were also used to quantitatively measure decorated areas in metaphase preparations and in interphase cells where chromosomal domains were well resolved. Quantitative evaluation of chromosome equivalents (Table 3) indicated highly concordant numbers for interphase versus metaphase in 5 of 6 examples; only in TC 593 decorated with 18 inserts was there a discrepancy. This may be due to the small sample size.

Table 3.

Twenty-four metaphase spreads showing the predominant number of chromosomes decorated with DNA inserts from libraries of chromosomes 4, 7 and 18 were compared to twenty-eight interphase nuclei with well-separated domains using the same probes. Images were taken under identical (linear film) conditions and digitized. In each metaphase spread, areas obtained for each normal and aberrant chromosome were divided by the mean area obtained for n apparently complete chromosomes. In interphase nuclei, domains were compared assuming that the largest n-labeled domains represented complete (normal) interphase chromosomes. Thus the sum of these normalized values represents a measure of the number of specific chromosomes equivalents in a single cell. The mean values of several cells are shown for each case. The mean numbers of chromosomes equivalents obtained for interphase and metaphase cells show a strong overall correlation coefficient of $r = + 0.95$. Compared with area measurements, the mean numbers of chromosomes equivalents determined by 2-D intensity integrals (See above) showed an overall correlation coefficient of $r = + 0.99$.

Table 3.

| Mean number of chromosome equivalents measured by digital image analysis in malignant glioma cell lines after CISS hybridization | | | | |
|---|---|---|---|---|
| Chromosome | Cell Line | Chromosome equivalents | | |
| | | Interphase | Methaphase | Expected |
| 4 | TC593 | 2.0 | 2.0 | 4.0 |
| | TC620 | 2.5 | 2.4 | 3.0 |
| 7 | TC593 | 4.4 | 4.6 | 4.0 |
| | TC620 | 3.5 | 3.3 | 3.0 |
| 18 | TC593 | 3.0 | 3.6 | 4.0 |
| | TC620 | 2.5 | 2.3 | 3.0 |

Chromosome equivalents derived from digital image analysis independently confirm the relative representation of target chromosomes noted in both glioma lines by DAPI banding. The segments that comprise the total metaphase signal are further detailed graphically in Fig. 13. Computer analysis was especially useful in cases where the breakpoints involved in translocated segments could not be unambiguously defined. They were also of value in a quantitative assessment of interphase-metaphase correlations, and of normal and aberrant chromosomes with distinctly different sizes.

EXAMPLE 3 Rapid Detection of Human Chromosome 21 Aberrations By In Situ Hybridization

DNA Probes

All plasmids contain inserts of human chromosome 21 that were mapped to 21q22.3. Moisan, J.P., Mattei, M.G., Baeteman-Volkel, M.A., Mattei, J.F., Brown, A.M.C., Garnier, J.M., Jeltsch, J.M., Masiakowsky, P., Roberts, M. & Mandel, J.L. (1985) Cytogenet. Cell Genet. 40, 701-702 (abstr.). Tanzi, R., Watkins, P., Gibons, K., Faryniarz, A., Wallace, M., Hallewell, R., Conneally, P.M. & Gusella, J. (1985) Cytogenet. Cell Genet. 40, 760 (abstr.). Van Keuren, M.L., Watkins, P.C., Drabkin, H.A., Jabs, E.W., Gusella, J.F. & Patterson, D. (1986) Am. J. Hum. Genet. 38, 793-804. Nakai, H., Byers, M.G., Watkins, P.C., Watkins, P.A. & Shows, T.B. (1987) Cytogenet. Cell Genet. 46, 667 (abstr.). Munke, M., Foellmer, B., Watkins, P.C., Cowan, J.M., Carroll, A.J., Gusella, J.F. & Fracke, U. (1988) Am. J. Humm. Genet. 42, 542-549. All inserts were either known or verified by Southern blot analysis to be single-copy DNA: the plasmids other than pS2 are subclones derived from a λ phage library or a cosmid library. Van Keuren, M.L., Watkins, P.C., Drabkin, H.A., Jabs, E.W., Gusella, J.F. & Patterson, D. (1986) Am. J. Hum. Genet. 38, 793-804. Masiakowski, P., Breathnach, R., Bloch, J., Gannon, F., Krust, A. & Chambon, P. (1982) Nucleic Acids. Res. 10, 7895-7903. Watkins, P.C., Tanzi, R.E. Gibbons, K.T., Tricoli, J.V., Landes, G., Eddy, R., Shows, T.B. & Gusella, J.F. (1985) Nucleic Acids Res. 13, 6075-6088. Watkins, P.C. Watkins, P.A., Hoffman, N. & Stanislovitis, P. (1985) Cytogenet. Cell Genet. 40, 773-774 (abstr.). The plasmids are listed in Table 4 with the Human Gene Mapping Workshop symbols and the approximate insert fragment length. Kaplan, J.C. & Carrit, B. (1987) Cytogenet. Cell Genet. 46, 257-276.

Table 4.

| Plasmids with inserts from 21q22.3 | | | | | |
|---|---|---|---|---|---|
| | Plasmid | Insert length kb | | Plasmid | Insert length kb |
| BCEI | pS2 (23) | 0.6 | D21S56 | pPW520-10R | 4.6 |
| D21S3 | pPW231F | 0.8 | | pPW520-11R | 1.8 |
| | pPW231G | 0.7 | D21S57 | pPW523-10B | 6.5 |
| D21S23 | pPW244D | 1.0 | | pPW523-1H | 7.0 |
| D21S53 | pPW512-6B | 3.0 | | pPW523-5R | 2.2 |
| | pPW512-8B | 3.8 | | pPW523-10R | 3.8 |
| | pPW512-1H | 2.9 | | pPW523-19R | 2.5 |
| | pPW512-16P | 2.7 | D21S64 | pPW551-8P | 1.9 |
| | pPW512-18P | 1.6 | | pPW551-12P | 4.2 |
| | pPW512-4R | 4.7 | D21S71 | pPW519-10P | 0.8 |
| | pPW512-12R | 2.0 | | pPW519-11P | 3.0 |
| D21S55 | pPW518-4H | 1.6 | | pPW519-1R | 6.0 |
| | pPW518-10P | 2.9 | | pPW519-8R | 2.9 |
| | pPW518-5R | 5.2 | | pPW519-9R | 1.7 |
| D21S56 | pPW520-5B | 5.0 | | pPW519-14R | 4.0 |
| | pPW520-6B | 1.0 | | pPW519-22R | 1.8 |

Preparation of plasmid DNA was according to standard protocols. Maniatis, T., Fritsch, E.F. & Sambrook, J.

(1982) Molecular Cloning: A Laboratory manual (Cold Spring Harbor Lab., Cold Spring Harbor, NY). Various probe sets were obtained by pooling plasmids (equal molar amounts), resulting in DNA probe complexities of 95 kb (all plasmids listed). 75 kb (plasmids labeled with an asterisk), or 29 kb (plasmids labeled with a dagger).

The human chromosome 21 genomic library LL21NSO2 was obtained from the American Type Culture Collection and amplified on agar plates as recommended. Phage DNA was prepared and digested wtih HindIII, and the DNA inserts were separated from the vector arms by preparative gel electrophoresis in 0.6% agarose. DNA was isolated from gel slices by electroelution; purified by Elutip-d chromatography. (Schleicher & Schuell); extracted with phenol/chloroform, 1:1 (vol/vol); and precipitated with ethanol.

Human Cells

Metaphase spreads and interphase nuclei were prepared from (i) lymphocyte cultures of normal (46, XY) individuals, (ii) lymphocytes of Down syndrome (47, +21) individuals, (iii) chorionic villi samples cultured for prenatal diagnosis (ii and iii were provided by T. Yan-Geng, Yale University Cytogenetics Laboratory), and (iv) cultures of TC620, an oligodendroglioma-derived pseudotriploid cell line. Manuelidis, L. & Manuelidis, E.E. (1979) in Progress in Neuropathology, ed. Zimmerman, H.M. (Raven, Press New York), Vol. 4, pp. 235-266. Standard techniques of colcemid treatment, hypotonic treatment, and methanol/acetic acid fixation were used. Biopsy material from the cortical region of a "normal" human brain (46, XX) was fixed, sectioned, and permeabilized as described. Manuelidis, L. & Borden, J., Chromosoma, 96:397-410(1988).

In situ Hybridization

Various combinations of plasmid DNA, labeled with BioII-dUTP by nick-translation, were used for hybridization at concentrations ranging from 2 to 15 μg/ml depending on the pool size. Brigati, D.J., Myerson, D. Leary, J.J., Spalholz, B., Travis, S.Z., Fong, C.K.Y. Hsiung, G.D. & Ward, D.C. (1983) Virology 126,32-50. For example, 15 μg/ml was used when the probe mixture contained 94 kilobases (kb) of insert DNA; the probe concentration was decreased in proportion to the sequence complexity of the probe mixture. The size of the probe DNA was adjusted to a length of 150-250 nucleotides empirically by varying the DNase concentration in the nick-translation reaction. The hybridization cocktail also contained 50% formamide, 0.30 M NaCl, 0.03 M sodium citrate (pH7), 10% (wt/vol) dextran sulfate, and on occasion 0.5 mg of sonicated salmon sperm DNA per ml. Simultaneous denaturation of probe and target DNA was carried out at 75°C for 6 min (metaphase spreads) or 94°C for 11 min (tissue slices). Hybridization reactions were incubated at 37°C overnight.

Delineation of individual chromosomes with DNA probes derived from sorted human chromosomes was done by (CISS) hybridization as described above. Briefly, biotinylated chromosome 21 library DNA inserts (5μg/ml), DNase-digested human genomic DNA (200μg/ml), and salmon sperm DNA (800μg/ml) were combined in the hybridization solution, heat-denatured, and partially prehybridized for 10-30 min at 37°C before application to a separately denatured specimen.

Posthybridization washes, detection of hybridized probe by using either alkaline phosphate-conjugated avidin or fluorescein isothiocyanate-conjugated avidin, and photographic conditions were as described in Example 1. When probe sets containing 29 kb or less of target sequence were used, the fluorescein isothiocyanate detection was generally enhanced by one cycle of signal amplification as described in Example 1.

All quantitative analyses of interphase signals were carried out by using slides from several independent experiments, with more than 100 nuclei being analyzed per slide. Comparison of signals in normal and trisomic samples was done in a blind-study fashion.

This work demonstrated that CISS hybridization, under the conditions described, resulted in rapid detection of numerical and structural aberrations of chromosome 21 in both metaphase and interphase cells.

Use of Cloned DNA Fragments From Human Chromosome 21 to Specifically Label Chromosomes in Lymphocyte Metaphase Spreads and Interphase Nuclei

The maximal amount of unique sequence DNA in the probe set was ca94 kb; this probe set resulted in a clearly visible labeling of the terminal region of both chromatids of the chromosome 21 homologs (see Fig. 14B). These signals were seen unambiguously and without exception in all metaphase spreads, even in spreads of poor quality or from prophase cells (not shown). In normal interphase cells, the majority (65-75%) of nuclei exhibited two signals (see Fig. 14C), 25-30% showed one signal, and less than 5% showed no signal. Nuclei with three signals were found only rarely (<0.2%) and may reflect incomplete hybridization to a few tetraploid cells in the sample. Similar results were obtained with probe sets containing 29 or 75 kb of DNA. With probe sets containing fewer than 20 kb of insert DNA, there were increased numbers of cells with less than two signals. Thus, these probe sets were deemed unsuitable for diagnostic purposes. However, such probes still yielded specific signals on the majority of chromosomes 21, even with a 6-kb single-copy DNA (see Fig. 14A), especially when signal amplification was used.

Use of chromosome library DNA CISS hybridization for detecting chromosome 21

Chromosome 21 was specifically and entirely decorated in normal lymphocyte metaphase spreads, although some additional minor binding sites were seen at or near the centromeric region of other acrocentric chromosomes, especially chromosome 13 (normal karyotype not shown; Fig. 14F). Suppression with additional DNA including a plasmid L1.26, which detects a repetitive DNA located predominantly at the centromeric region of chromosomes 13 and 21, did not efficiently suppress the minor non-21 chromosomal signals. Devilee, P., Cremer, T., Slagboom, P., Bakker, E., Schoil, H.P., Hager, H.D. Stevenson, A.F.G., Cornelisse, C.J. & Pearson, P.L. (1986) Cytogenet. Cell Genet. 41,193-201. Quantitative evaluation of interphase nuclei signals again showed a negligible portion of nuclei with three signals; however, a significant increase in nuclei with less than two signals was observed (50-60% with two signals, 35-45% with one signal, and 5-10% without a signal). The numerical differences observed with the two different probes can be explained in part by the number of nuclei (up to one of three) that were excluded from the latter analysis because they exhibited larger and more diffuse signals, most likely from more than one chromosome that could not be resolved unambiguously as two separate chromosome domains in a two-dimensional representation. The minor cross-hybridizing sites noted above presented a second experimental complication but did not adversely influence data interpretation.

Testing of Cells Containing Chromosome 21 Aberrations

The optimal (94 kb) plasmid pool as well as CISS hybridization with chromosome 21 library inserts were tested further by using cells containing chromosome 21 aberrations. Both probe sets permitted a fast and unambiguous diagnosis of trisomy 21 in all metaphase spreads from Down syndrome lymphocyte cultures (see examples in Fig. 14 D and E). Furthermore, the quantitative distribution of hybridization signals in interphase nuclei of the same preparation, analyzed as described above, was similar with either type of probe [<5% of cells with no signal, 5-15% with one signal, 25-35% with two signals, and 55-65% with three signals (Fig. 14 F-J)]. Although the library DNA inserts gave up to 15% of four-signal nuclei (compare Fig. 14 F and G), most likely due to the minor binding sites on other chromosomes, the plasmid pool revealed only a negligible percentage of nuclei (<0.2%) with four signals. These results indicate that trisomy 21 can be detected in a diagnostically meaningful way with small populations of nonmitotic cells.

Localization of Chromosome 21 DNA in Embryonic Chorionic Villi Cells

Embryonic chorionic villi (CV) cells were also investigated with the 94 kb plasmid probe sets in a case where the father had a reciprocal t(4:21) translocation. Hybridization to metaphase spreads of the CV cells showed that the translocated chromosome (4pter→4q33::21q11.2→21qter) was indeed inherited by the fetus (see Fig. 13 L and M). The signals in the interphase cell nuclei (see Fig. 14K) of the CV cells had a distribution that paralleled that of cells with a normal karyotype (see above), indicating a balanced representation of 21q22.3 and excluding Down syndrome as a possible diagnosis. A small increase of nuclei with three and four signals (both <5%) over that of normal lymphocytes was also observed, probably reflecting a higher portion of tetraploid cells in such CV samples.

Localization of Chromosome 21 DNA in of Glioma Tumor Cells

The diagnostic potential of the chromosome 21 probes was further tested by using a glioma tumor cell line, TC620, known to be pseudotriploid with a highly rearranged genome. Cremer, T. et al., Exp. Cell Res., 176:199-220 (1988); Cremer, T. et al., Hum. Genet., In Press, (1988); Manuelidis, L. and E.E. Manuelidis, In: Progress in Neuropathology, 4:235-266 (ed. Zimmerman, H.M.) (1979). The metaphase spreads revealed two apparently normal chromosomes 21 and one translocation chromosome (see Fig. 14 N and O). Interestingly, the chromosome 21 DNA on the translocation chromosome labeled by the library probe has a size equivalent to a normal 21q region, thus suggesting a Robertsonian translocation event. However, fine structural aberrations of 21q (i.e., small deletions, etc.) cannot be excluded by this analysis. The interphase signals seen with both the plasmid probe set and the library inserts were consistent with trisomy 21q22.3 and trisomy 21, respectively.

Localization of Chromosome 21 in DNA Sequences in Solid Tissues

The ability of the 94 kb plasmid probe set to localize chromosome 21 DNA sequences in solid tissues was also assessed. Both chromosomes 21 were clearly labeled by the probe, and located near the nucleolus; this nuclear location is consistent with the fact that chromosome 21 contains a ribosomal gene cluster that is usually localized in the nucleolus. This observation suggests that these probes may also prove useful for evaluating the frequency of chromosome 21 mosaicism in specific cell or tissue types. In addition, it should be of interest to see if the various karyotypic changes associated with the Down syndrome phenotype alter the normal nuclear topography of chromosome 21 in neuronal tissue.

**Claims**

1. A method for specifically staining or labelling any selected individual target chromosome(s) (or subregion(s) thereof) *in situ* in a sample containing chromosomal DNA from interphase cells by *in situ* hybridization in interphase cells or nuclei, comprising the steps of

(i) treating the sample to render the chromosomal DNA available for hybridization with complementary nucleic acid sequences,

(ii) hybridizing labelled probe DNA with the DNA of step (i) whereby hybridization of the probe DNA with ubiquitous repeated DNA sequences present in the DNA of step (i) (e.g. Alu and KpnI elements) is suppressed, suppression being achieved by hybridizing (e.g. by preannealing) competitor DNA which contains sequences that hybridize to the ubiquitous repeated DNA sequences with the probe DNA; wherein the probe DNA comprises DNA fragments smaller than 500 nucleotides in length.

2. The method of claim 1 for specifically staining or labelling a plurality of selected chromosomes (or subregions thereof), wherein more than one probe, each specific for a chromosome or subregion thereof and each labelled with a distinct reporter molecule, is provided.

3. The method of claim 1 or claim 2 wherein the interphase cells are tumour cells or uncultured cells from amniotic fluid.

4. The method of any one of the preceding claims, wherein the probe DNA is obtained from a chromosome-derived library.

5. The method of any one of the preceding claims, wherein the probe DNA and the competitor DNA comprise DNA fragments smaller than 500 nucleotides, for example 150-250 nucleotides in length.

6. The method of any one of the preceding claims, wherein the competitor DNA for suppression of repetitive sequences is total DNA, for example total human genomic DNA.

7. The method of any one of the preceding claims, wherein step (ii) is conducted in the presence of a carrier DNA.

8. The method of any one of the preceding claims wherein the probe DNA is labelled with: (a) avidin or biotin, (b) a fluorochrome or fluorochrome combination, or (c) an enzyme.

9. The method of claim 8(b) wherein the fluorochrome(s) are selected from fluorescein, rhodaimine, Texas red, Lucifer yellow, a phycobiliprotein and a cyanin dye.

**Patentansprüche**

1. Ein Verfahren zum spezifischen Anfärben oder Markieren eines beliebig ausgewählten individuellen Zielchromosoms oder individueller Zielchromosomen (oder eines Teilbereichs oder Teilbereichen davon) in situ in einer Probe, die chromosomale DNS aus Interphasezellen enthält, durch in situ Hybridisierung in Interphasezellen oder Kernen, umfassend die Schritte:

(i) Behandlung der Probe, um die chromosomale DNS für eine Hybridisierung mit komplementären Nucleinsäuresequenzen verfügbar zu machen,

(ii) Hybridisierung markierter Sonden-DNS mit der DNS aus Schritt (i), wodurch eine Hybridisierung der Sonden-DNS mit ubiquitären repetitiven DNS-Sequenzen, die in der DNS aus Schritt (i) (z. B. Alu und KpnI Elemente) vorhanden sind, unterdrückt wird, wobei die Unterdrückung durch Hybridisierung (z.B. durch Vortempern) einer Kompetitor-DNS erreicht wird, die Sequenzen enthält, die an die ubiquitären repetitiven DNS-Sequenzen mit der Sonden-DNS hybridisieren; worin die Sonden-DNS DNS-Fragmente umfaßt, die längenmäßig kleiner sind als 500 Nukleotide.

2. Das Verfahren nach Anspruch 1 zum spezifischen Anfärben oder Markieren einer Vielzahl ausgewählter Chromosomen (oder Teilbereichen davon) , worin mehr als eine Sonde bereitgestellt wird, wobei jede spezifisch für ein Chromosom oder Teilbereich davon ist und jede mit einem verschiedenen Reporter-Molekül markiert ist.

3. Das Verfahren nach Anspruch 1 oder Anspruch 2, worin die Interphasezellen Tumorzellen oder nichtkultivierte Zellen aus Amnionflüssigkeit sind.

4. Das Verfahren nach einem der vorhergehenden Ansprüche, worin die Sonden-DNS aus einer von Chromosomen abstammenden Bibliothek erhalten ist.

5. Das Verfahren nach einem der vorhergehenden Ansprüche, worin die Sonden-DNS und die Kompetitor-DNS DNS-Fragmente umfassen, die längenmäßig kleiner sind als 500 Nucleotide, zum Beispiel 150-250.

6. Das Verfahren nach einem der vorhergehenden Ansprüche, worin die Kompetitor-DNS zur Unterdrückung von repetitiven Sequenzen eine Gesamt-DNS, zum Beispiel menschliche genomische Gesamt-DNS, ist.

7. Das Verfahren nach einem der vorhergehenden Ansprüche, worin Schritt (ii) in Gegenwart einer Carrier-DNS durchgeführt wird.

8. Das Verfahren nach einem der vorhergehenden Ansprüche, worin die DNS-Sonde markiert ist mit: (a) Avidin oder Biotin, (b) einem Fluorochrom oder einer Fluorochrom-Kombination, oder (c) einem Enzym.

9. Das Verfahren nach Anspruch 8(b), worin das/die Fluorochrom(e) ausgewählt ist/sind aus Fluorescein, Rhodamin, Texas Rot, Lucifergelb, einem Phycobiliprotein- und einem Cyanin-Farbstoff.

## Revendications

1. Méthode pour spécifiquement colorer ou marquer *in situ* tout chromosome cible individuel sélectionné (ou une (des) sous-région(s) de celui-ci) dans un échantillon contenant de l'ADN chromosomique de cellules en interphase par hybridation *in situ* des noyaux ou cellules en interphase comprenant les étapes consistant à :

   (i) traiter l'échantillon pour rendre l'ADN chromosomique disponible pour une hybridation avec des séquences d'acides nucléiques complémentaires ;
   (ii) réaliser l'hybridation d'une sonde marquée avec l'ADN de l'étape (i) en supprimant l'hybridation de la sonde d'ADN avec des séquences d'ADN répétées ubiquitaires présentes dans l'ADN de l'étape (i) (exemple : des éléments Alu et KpnI), la suppression étant réalisée en hybridant (exemple : par précircularisation) de l'ADN compétiteur qui contient des séquences qui hybrident aux séquences d'ADN répétées ubiquitaires avec la sonde d'ADN, ladite sonde d'ADN comprenant des fragments d'ADN de taille inférieure à 500 nucléotides.

2. Méthode selon la revendication 1, pour spécifiquement colorer ou marquer une pluralité de chromosomes sélectionnés (ou des sous-régions de ceux-ci) dans laquelle on dispose de plus d'une sonde, chacune étant spécifique pour un chromosome ou une sous-région de celui-ci et chacune étant marquée avec une molécule traceur distincte.

3. Méthode selon la revendication 1 ou 2, dans laquelle les cellules en interphase sont des cellules tumorales ou des cellules non cultivées provenant de liquide amniotique.

4. Méthode selon l'une quelconque des revendications précédentes, dans laquelle la sonde d'ADN est obtenue à partir d'une banque dérivée de chromosomes.

5. Méthode selon l'une quelconque des revendications précédentes, dans laquelle la sonde d'ADN et l'ADN compétiteur comprennent des fragments d'ADN inférieurs à 500 nucléotides, par exemple, d'une longueur de 150 à 250 nucléotides.

6. Méthode selon l'une quelconque des revendications précédentes, dans laquelle l'ADN compétiteur pour la suppression des séquences répétitives est de l'ADN total, par exemple de l'ADN génomique humain total.

7. Méthode selon l'une quelconque des revendications précédentes, dans laquelle l'étape (ii) est réalisée en présence d'un ADN porteur.

8. Méthode selon l'une quelconque des revendications précédentes, où la sonde d'ADN est marquée avec : (a) l'avidine ou la biotine, (b) un fluorochrome ou une combinaison de fluorochromes, ou (c) une enzyme.

9. Méthode selon la revendication 8(b) dans laquelle le(s) fluorochrome(s) est(sont) choisi(s) parmi la fluorescéine, la rhodamine, le rouge Texas, le jaune Lucifer, une phycobiliprotéine et un colorant à base de cyanine.

```
┌──────────┐        ┌─────────────────────────────────┐        ┌──────────────────┐
│          │        │ Probe DNA with detectable label  │        │ Competitor DNA   │
│  Sample  │        │ chromosomal DNA fragments        │        │ (from,e.g.       │
│          │        │ (from genetic DNA library or cloned│  +   │ WBC placental)   │
│          │        │ DNA fragments)                   │        │                  │
└──────────┘        │  *concentration adjusted to reflect│      └──────────────────┘
                    │   relative DNA content of        │
                    │   chromosome target              │
                    └─────────────────────────────────┘
```

Denature
DNA

Add carrier DNA to adjust final DNA concentration
Heat to denature
Incubate to initiate partial reannealing

Place on pre·
warmed slide

```
┌──────────────────┐        ┌─────────────────────────┐
│ Sample with      │        │                         │
│ denatured DNA    │   +    │ Hybridization mixture   │
└──────────────────┘        └─────────────────────────┘
```

```
┌────────────────────────────────────────────────────────┐
│ Combine sample with      Hybridization mixture. Seal    │
│ denatured DNA       +    and incubate at 37° C under    │
│                          moist conditions               │
└────────────────────────────────────────────────────────┘
```

Wash/Detect hybridized/labeled sample DNA

*Fig. 1* OUTLINE OF CISS HYBRIDIZATION FOR SPECIFIC
STAINING OF HUMAN CHROMOSOMES

EP 0 444 115 B2

# FIG.2

# FIG.3

# FIG.4

# FIG.5

# FIG.6

# FIG.7

FIG.8

# FIG.9

FIG.IO

FIG. 11A

FIG. 11B

FIG. 11C

FIG. 11D

FIG. 11E

FIG.12

FIG. 13

# FIG.14